(19) Europäisches Patentamt / European Patent Office / Office européen des brevets

(11) **EP 2 723 885 B1**

(12) **FASCICULE DE BREVET EUROPEEN**

(45) Date de publication et mention de la délivrance du brevet:
**12.08.2015 Bulletin 2015/33**

(21) Numéro de dépôt: **12745547.5**

(22) Date de dépôt: **27.06.2012**

(51) Int Cl.:
***C12Q 1/04*** (2006.01) ***G01N 21/17*** (2006.01)
***G01N 21/41*** (2006.01) ***G01N 21/64*** (2006.01)

(86) Numéro de dépôt international:
**PCT/IB2012/053249**

(87) Numéro de publication internationale:
**WO 2013/001465 (03.01.2013 Gazette 2013/01)**

(54) **PROCÉDÉ POUR CARACTÉRISER LA PRÉSENCE OU L'ABSENCE D'UN MICROORGANISME, DANS UN MILIEU BIOLOGIQUE**

VERFAHREN ZUR KLASSIFIZIERUNG DER AN- ODER ABWESENHEIT EINES MIKROORGANISMUS IN EINEM BIOLOGISCHEN MEDIUM

METHOD FOR CLASSIFYING THE PRESENCE OR ABSENCE OF A MICROORGANISM IN A BIOLOGICAL MEDIUM

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **27.06.2011 FR 1155690**

(43) Date de publication de la demande:
**30.04.2014 Bulletin 2014/18**

(73) Titulaires:
- **Commissariat à l'Énergie Atomique et aux Énergies Alternatives 75015 Paris (FR)**
- **Centre National de la Recherche Scientifique 75794 Paris Cedex 16 (FR)**

(72) Inventeurs:
- **MARCOUX, Pierre F-38120 Saint Egreve (FR)**
- **DUPOY, Mathieu F-38100 Grenoble (FR)**
- **GUILLEMOT, Laure-Hélène F-75017 Paris (FR)**
- **TRAN-THI, Thu-Hoa F-92120 Montrouge (FR)**

(74) Mandataire: **Nony 3, rue de Penthièvre 75008 Paris (FR)**

(56) Documents cités:
**WO-A1-94/04705** **WO-A2-2010/004225**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

La présente invention vise à proposer un procédé utile pour détecter la présence d'au moins un microorganisme, généralement pathogène, notamment dans un échantillon biologique, de préférence physiologique, et plus particulièrement de sang.

L'hémoculture est la culture du sang dans ou sur un milieu nutritif afin d'obtenir une croissance bactérienne. L'objectif consiste à amplifier la concentration d'une espèce pathogène en lui fournissant des conditions favorables de croissance, de manière à la détecter chez un patient présentant un état septique.

L'hémoculture est actuellement la seule méthode de détection des infections sanguines et elle représente, pour les laboratoires de microbiologie, un test fréquent, de l'ordre du tiers de l'activité.

A la base, il s'agit d'un simple test de détection. La technique manuelle, conventionnelle, consiste à incuber des flacons renfermant l'échantillon biologique à analyser dans une étuve, entre 35 et 37 °C, et à observer chaque flacon une fois par jour à la recherche d'une prolifération bactérienne sous forme d'un trouble, et cela, pendant un minimum de 10 jours.

Les avancées technologiques des trente dernières années dans le domaine de l'hémoculture ont permis l'automatisation des analyses, ainsi qu'une réduction significative du temps de détection et d'identification.

L'automatisation de ce procédé consiste généralement à détecter un métabolite, c'est-à-dire un composé chimique issu du métabolisme bactérien, une sorte de déchet inhérent à la croissance microbienne. Bon nombre de ces métabolites, dits encore ci-après VOC (Volatil Organic Compound ou COV, Composé Organique Volatil), sont gazeux ou volatils. Ainsi, des automates exploitent la formation de $CO_2$, qui se révèle être le métabolite le plus générique, émis par des micro-organismes en croissance. A l'inverse, d'autres automates peuvent révéler une croissance microbienne en détectant une consommation de nutriment, comme la consommation d'$O_2$ par exemple.

Pour ce qui est de la détection du $CO_2$, plusieurs technologies se sont succédées, se distinguant notamment par le mode d'analyse retenu pour la caractérisation du $CO_2$.

Développée à partir des années 1970, une technologie dite BACTEC en continu, avec une détection non-invasive, est basée sur la respirométrie radiométrique. Plus précisément, elle repose sur la métabolisation de substrats enzymatiques marqués $^{14}C$ de manière à permettre l'émission d'un métabolite volatil détecté par comptage beta à 156 keV.

Selon une de ses variantes de réalisation, dite BACTEC 9240, le fond d'un flacon renfermant l'échantillon biologique à caractériser, contient un polymère généralement siliconé, perméable aux gaz, et incluant une molécule sonde fluorophore sensible au pH. Lorsque le $CO_2$ diffuse du liquide vers le matériau hydraté, il forme de l'acide carbonique $H_2CO_3$ qui acidifie le polymère et accroît la fluorescence émise par le fluorophore (EP 682 252).

Une autre technologie, dite BacT/ALERT, très voisine de celle du BACTEC 9240, met en oeuvre un polymère perméable aux gaz incluant également une molécule sonde sensible au pH, mais figurée, dans ce cas, par une sonde chromophore. Lorsque le $CO_2$ diffuse du liquide vers le matériau hydraté, il forme de l'acide carbonique $H_2CO_3$ qui acidifie le polymère, et le rend moins opaque et plus réfléchissant.

Dans les années 1990, il a été proposé un automate d'hémoculture à suivi continu (VITAL), basé également sur l'emploi de molécules sondes fluorescentes en vue d'une caractérisation par transduction optique. Les sondes sont toutefois disposées, non pas au fond du flacon, mais en solution dans le milieu de culture. Leur fluorescence est inhibée par les protons et les molécules réductrices. Par conséquent, la croissance bactérienne se révèle par une baisse de fluorescence.

Un autre type de technologie, non spécifique à la détection d'un VOC en particulier, et distribué sous le nom de Versa TREK, repose sur une détection manométrique. C'est l'ensemble des métabolites gazeux générés, dont majoritairement le $CO_2$, qui est détecté. Chaque flacon est fermé hermétiquement et équipé d'un détecteur qui suit en continu la pression du gaz au dessus du milieu de culture.

Il a également été proposé des dispositifs permettant de détecter simultanément plusieurs métabolites volatils en phase gazeuse. Un tel dispositif comprend plusieurs détecteurs qui sont respectivement spécifiques à un VOC particulier (ou à une famille particulière de VOC), et la détection se fait à l'aide d'une transduction optique directe. Un substrat est imprégné d'un "indicateur", c'est-à-dire une molécule capable de subir un changement de couleur en présence du VOC identifié comme pertinent (JG McDonald et al.: WO 2006/107370).

C'est une phase exploratoire d'analyse des VOC émis par une espèce donnée qui permet ultérieurement de caractériser dans un milieu à analyser la présence des mêmes VOC. Les VOC émis par une culture de cette espèce sont concentrés par adsorption sur une cartouche de SPME. Les composés sont ensuite désorbés et analysés par couplage GC-MS. La chromatographie en phase gaz (GC) permet une quantification des différents composés et la spectrométrie de masse (MS) est utilisée à profit pour leur identification.

Une autre technologie propose la mise en oeuvre d'une grande variété de sondes susceptibles de réagir avec des VOC par interactions non covalentes (interactions de Van der Waals, liaisons hydrogènes, complexes $\pi$-$\pi$, transfert de charge, interactions acide-base au sens de Brönsted ou de Lewis). Un VOC cible va interagir avec une ou des

cibles(s) ce qui va entraîner un ou des changement(s) de couleur (K.S. Suslick et al.: US 2003/0166298). La cartographie des changements de couleur de la matrice de sondes constitue la réponse multiparamétrique du détecteur à la présence du VOC.

Ce principe d'identification par colorimétrie est notamment mis à profit dans le domaine du diagnostic *in vivo* non invasif. Il est plus précisément utilisé pour explorer la présence d'un pathogène dans l'estomac (*Helicobacter pylori*) grâce à une analyse de l'haleine du sujet. *H. pylori* se caractérise par une forte production d'uréase, une enzyme qui dégrade l'urée en ammoniac et $CO_2$. Une pastille d'urée est donnée au patient dont l'haleine est ensuite mise en contact avec une matrice de sondes colorimétriques.

Pour ce qui est de la détection des mycobactéries, les automates sont basés sur les mêmes technologies que celles des automates pour hémoculture. En effet, l'objectif est le même, à savoir détecter une croissance microbienne dans un milieu de culture liquide. La seule différence réside dans la composition des milieux de culture.

En fait, comme il ressort de ce qui précède, l'ensemble des technologies discutées ci-dessus repose pour l'essentiel sur la caractérisation d'un métabolite inhérent à la croissance microbienne et généralement non spécifique d'un microorganisme, tel que le métabolite $CO_2$. La formation de ce métabolite est généralement sanctionnée *via* son interaction avec une sonde fluorophore sensible au pH ou une sonde chromophore disposée à cet effet dans l'enceinte contenant le milieu à analyser.

Il apparait que les modes de détection précités ne s'avèrent pas totalement satisfaisants.

Ainsi, en ce qui concerne la détection du $CO_2$ par la technique de BioLumix (US 2010/0273209 A1), par exemple, il existe un fond élevé pour le $CO_2$, notamment le $CO_2$ résiduel dans l'air (≈390 ppm), qui perturbe les résultats.

Dans les technologies BacT/ALERT (J. Turner et al., US 4,945,060) ou BioLumix (US 2010/0273209 A1), les sondes employées sont des sondes de pH. Ces sondes ne sont donc pas spécifiques. Tout autre métabolite volatile présentant des propriétés acido-basiques (accepteurs ou donneurs de protons, comme l'ammoniac ou l'indole par exemple) va, si présent, perturber les résultats. Dans les milieux tamponnés, comme le sang par exemple, la détection de l'acide carbonique est alors plus difficile.

Par ailleurs, ces technologies emploient des supports développant une faible surface massique (en $m^2/g$). Or, il s'agit d'une réaction hétérogène. La sonde est en effet à la surface d'un solide, et le métabolite cible est dans un fluide (liquide ou gaz) au contact du détecteur. Pour améliorer les cinétiques de détection, il apparaît donc important d'augmenter au maximum la surface de contact entre la phase solide et le fluide.

De plus, dans la technologie de BacT/ALERT par exemple, la distance de diffusion que doit parcourir dans certains cas le métabolite volatil est importante (US 4,945,060).

Enfin, ces technologies ne permettent pas, si nécessaire, de s'affranchir de l'effet parasitaire notamment en termes de fluorescence, coloration ou encore absorbance, susceptible d'être généré parallèlement par des composés annexes et donc distincts du microorganisme à caractériser et qui seraient présents également dans le milieu.

En conséquence, la présente invention vise à proposer un nouveau mode de détection permettant de suppléer au moins en partie aux inconvénients précités.

Ainsi, est décrit un procédé pour révéler la présence ou l'absence d'au moins un microorganisme dans un milieu biologique, ledit procédé comprenant au moins les étapes consistant à :

1) disposer d'une enceinte contenant :

- une phase liquide ou semi-solide formée en tout ou partie dudit milieu biologique susceptible de contenir au moins une forme vivante dudit microorganisme, des éléments nutritifs nécessaires à la prolifération dudit microorganisme, et d'un substrat enzymatique spécifique audit microorganisme et métabolisable en au moins un métabolite volatil VOC, et
- une phase gazeuse contiguë à ladite phase liquide ou semi-solide,

2) exposer au moins ladite phase liquide ou semi-solide à des conditions propices à la métabolisation dudit substrat enzymatique par ledit microorganisme, en au moins une molécule dudit métabolite VOC, et

3) révéler, par transduction optique, la présence ou l'absence dudit métabolite VOC, indicateur de la présence dudit microorganisme,

caractérisé en ce que ledit métabolite VOC, si formé en étape 2), interagit avec une matrice nanoporeuse, et en ce que la détection par transduction optique d'un changement des propriétés optiques de ladite matrice, est indicatrice d'une interaction de ladite matrice avec ledit métabolite.

La présente invention concerne un procédé pour révéler la présence ou l'absence d'au moins un microorganisme dans un milieu biologique, ledit procédé comprenant au moins les étapes consistant à :

1) disposer d'une enceinte contenant :

- une phase liquide ou semi-solide formée en tout ou partie dudit milieu biologique susceptible de contenir au moins une forme vivante dudit microorganisme, des éléments nutritifs nécessaires à la prolifération dudit microorganisme, et d'un substrat enzymatique spécifique audit microorganisme et métabolisable en au moins un métabolite volatil VOC, et
- une phase gazeuse contiguë à ladite phase liquide ou semi-solide,

2) exposer au moins ladite phase liquide ou semi-solide à des conditions propices à la métabolisation dudit substrat enzymatique par ledit microorganisme, en au moins une molécule dudit métabolite VOC, et
3) révéler, par transduction optique, la présence ou l'absence dudit métabolite VOC, indicateur de la présence dudit microorganisme,

caractérisé en ce que :

- ledit métabolite VOC, si formé en étape 2), interagit avec une matrice nanoporeuse, ladite matrice étant mise en oeuvre sous une forme séparée dudit substrat enzymatique, et
- la détection par transduction optique d'un changement des propriétés optiques de ladite matrice, est indicatrice d'une interaction de ladite matrice avec ledit métabolite.

Dans ce mode de réalisation, la matrice est disposée de manière à ne pas avoir de contact physique avec le ou les substrats enzymatiques également mis en oeuvre dans le procédé selon l'invention. Cette absence de tout contact entre le ou les capteur(s) présent au niveau de la matrice et l'analyte, permet avantageusement d'éviter tout phénomène de contamination, notamment de pollution de ce ou ces capteur(s) par l'analyte.
Ce défaut de contact entre les deux types d'entités peut être notamment obtenu en disposant en phase gazeuse la matrice. Toutefois, d'autres modes de séparation, notamment dans la variante ou la matrice est immergée, ou au contact de la phase liquide ou semi-solide contenant le substrat enzymatique, peuvent également être considérés. Leur mise au point relève clairement des compétences de l'homme de l'art.
Comme il ressort de ce qui précède, le procédé selon l'invention repose sur la détection d'un ou plusieurs microorganismes, notamment d'une ou plusieurs bactérie(s), dans un échantillon généralement biologique *via* la caractérisation, en phase gazeuse ou en phase liquide, d'une de leurs voies enzymatiques.
Plus précisément, pour la détection d'un ou plusieurs microorganisme(s), les enzymes de l'espèce du microorganisme présumé présent dans le milieu à analyser, conduisent à la dégradation du substrat enzymatique métabolisable considéré selon l'invention en au moins un métabolite volatil dit VOC. Les molécules gazeuses de ce métabolite VOC sont captées et concentrées dans une matrice nanoporeuse à grande surface spécifique et c'est le changement des propriétés optiques de la matrice nanoporeuse, induit par l'adsorption et l'accumulation du métabolite VOC dans ses pores, qui va permettre de révéler la présence ou non du microorganisme recherché dans le milieu biologique à analyser.
Selon un mode de réalisation préféré, ledit métabolite est doté de propriétés optiques intrinsèques.
Comme détaillé ci-après, ce métabolite possède en outre au sein de ladite phase liquide ou semi-solide une constante de Henry privilégiant un état gazeux.
Ce métabolite est avantageusement spécifique ou dit encore exogène.
Par le terme « métabolite VOC exogène », on entend un métabolite non naturellement généré lors du développement microbien du microorganisme considéré, ou encore distinct des métabolites naturels générés lors d'une croissance microbienne tel que par exemple le $CO_2$. Autrement dit, ce métabolite VOC n'est pas susceptible d'être généré par d'autres microorganismes que celui ou ceux spécifiquement recherché(s).
Ainsi, selon un mode de réalisation préféré, ledit métabolite VOC considéré selon l'invention est distinct des métabolites naturels générés lors d'une croissance microbienne.
Avantageusement, le métabolite VOC considéré selon l'invention est un métabolite spécifique à la métabolisation du substrat enzymatique associé et est donc représentatif d'une voie enzymatique sélective au microorganisme recherché.
La matrice nanoporeuse considérée selon l'invention peut être disposée en phase gazeuse ou en phase liquide dans ladite enceinte.
Dans une variante de réalisation préférée, la détection par transduction optique peut être avantageusement réalisée au niveau de ladite matrice qui est disposée en phase gazeuse.
Ce mode de réalisation permet de s'affranchir de l'effet parasitaire notamment en terme de fluorescence, coloration ou encore absorbance, susceptible d'être généré parallèlement par des composés annexes et donc distincts du microorganisme à caractériser et qui seraient présents également en phase liquide.
Dans un tel mode de réalisation, la matrice localisée dans la phase gazeuse, peut-être par exemple disposée dans le volume mort d'un flacon d'hémoculture, *headspace*.
Selon un mode de réalisation spécifique, la matrice nanoporeuse comprend une molécule, dite molécule sonde,

susceptible d'interagir spécifiquement avec le VOC que l'on cherche à détecter. Cette interaction spécifique entraîne la formation d'un produit dont les propriétés optiques sont détectables. Ce mode de réalisation permet généralement une meilleure spécificité de la détection.

On entend par « milieu biologique », tout échantillon de matière, liquide, semi-solide, ou solide, susceptible de convenir à la survie, voire à la croissance et/ou la prolifération d'au moins un microorganisme.

A titre d'exemple non-limitatif, un milieu biologique peut être un milieu dédié à une application alimentaire, cosmétique, pharmaceutique et plus préférentiellement être un milieu physiologique.

Plus particulièrement, un milieu biologique peut notamment être un échantillon biologique tel que le sérum, l'urine, la salive ou le sperme, un échantillon très coloré (boissons colorés, sang) ou diffusant (matrices alimentaires) ou intrinsèquement fluorescent (certains milieux de culture spécifique, comme le Löwenstein-Jensen, ou certaines matrices alimentaires comme le foie).

De préférence, le milieu biologique est formé en tout ou partie d'un échantillon alimentaire ou d'un échantillon physiologique.

Un microorganisme considéré par l'invention peut être figuré par une espèce de bactérie, de levure, de champignon, de protozoaire, voire de virus cultivé dans une cellule hôte. De préférence, un microorganisme de l'invention est figuré par une espèce de bactérie, notamment susceptible d'être pathogène pour un mammifère tel que l'homme.

La phase liquide ou semi-solide considérée dans son ensemble représente le milieu de culture de microorganisme à détecter.

Par le terme « semi-solide », on entend notamment désigner les milieux de culture dits gélosé.

Comme détaillé ci-après, la détection du métabolite VOC peut être réalisée en absorbance et/ou en fluorescence.

Selon un mode de réalisation préféré, la matrice nanoporeuse est utilisée comme guide d'onde et possède par exemple une forme de cylindre allongé. Avec un indice plus élevé que l'air ou l'eau, ce guide d'onde peut être plongé dans la phase fluide (liquide ou gaz) où se trouve le métabolite cible.

Une géométrie de guide d'onde en forme de cylindre allongé peut permettre de réduire la distance que doit parcourir le métabolite cible avant d'atteindre le faisceau lumineux.

Le procédé considéré selon l'invention est avantageux à plusieurs titres.

Tout d'abord, le fait que le métabolite volatil VOC libéré est adsorbé en phase gazeuse ou en phase liquide dans une matrice poreuse, de préférence transparente, et avantageusement à grande surface spécifique, c'est à dire au moins égale à 300 $m^2/g$ et de préférence entre 400 et 900 $m^2/g$, permet d'accroître significativement la cinétique de détection comparativement aux technologies conventionnelles.

Le procédé selon l'invention est en outre compatible avec une caractérisation de microorganismes cultivés dans tous types de milieux, c'est-à-dire qu'ils soient liquides ou non, colorés ou non, diffusants ou non, comme par exemple les hémocultures (forte absorbance de l'hémoglobine du sang, à laquelle il faut ajouter la lumière diffusée et absorbée par les particules inhibant les antibiotiques), et certaines matrices alimentaires (additifs alimentaires avec fluorescence intrinsèque ou milieux diffusants) ou encore certains milieux de cultures pour microorganismes.

Dans tous ces milieux, l'emploi de substrats enzymatiques conventionnels, chromogènes ou fluorogènes, est en effet d'ordinaire perturbé par les propriétés optiques intrinsèques de ceux-ci. Par exemple, le substrat p-nitrophényl-β-D-galactopyranoside (pNPG) est connu depuis longtemps pour mettre en évidence la présence de bactéries, par suivi de l'absorbance de la phase liquide associée à 301 nm ou à 418 nm. Malheureusement, les technologies d'analyse reposant sur l'emploi d'un tel substrat chromogène, ne sont pas applicables dans un milieu de culture diffusant et/ou absorbant.

Cet avantage est également particulièrement appréciable pour la détection des mycobactéries. En effet, les milieux de culture employés classiquement pour la caractérisation de ces microorganismes, doivent comporter des inhibiteurs dans le souci d'éviter que d'autres microorganismes, dus à une contamination de l'échantillon, ne s'y développent au détriment des mycobactéries que l'on cherche à détecter. Or, pour l'essentiel, ces inhibiteurs sont à la fois colorés et fluorescents, ce qui entrave l'emploi de substrats chromogènes ou fluorogènes pour la mise en évidence des mycobactéries potentiellement présentes dans la culture.

Certes, le principe de détecter un microorganisme *via* la caractérisation d'un VOC figurant un produit de métabolisme inhérent à sa croissance et sa prolifération, à l'image notamment du $CO_2$, par transduction optique est connu, ce principe est notamment décrit dans WO 2006/107370, US 2005/0171449, WO 2010/028057, US 2003/0166298 et US 2008/0199904. Toutefois, l'ensemble des technologies existantes requiert généralement l'usage de sonde(s) dédiée(s) à interagir avec un VOC formé lors du développement microbien, généralement le $CO_2$, et ne sont donc pas spécifiques à une voie enzymatique particulière et représentative d'un microorganisme spécifique.

Les technologies de l'art antérieur ne mettent donc pas en oeuvre de substrat spécifique à une voie enzymatique.

Par ailleurs, lorsque ces sondes sont supportées, les matériaux considérés dans ces technologies conventionnelles, ne permettent pas d'accéder à une sensibilité de détection satisfaisante, au regard de leur faible surface spécifique.

Par opposition, l'invention a pour avantage la mise en oeuvre d'une interaction spécifique entre la matrice

nanoporeuse, ou la molécule sonde qu'elle contient, et un VOC, ce dernier étant représentatif d'une voie enzymatique spécifique au microorganisme recherché. Ainsi, pour caractériser une enzyme particulière, on peut déterminer un substrat dit spécifique à une voie enzymatique, de façon à ce que l'interaction entre l'enzyme et le substrat génère un VOC prédéterminé, aisément détectable.

Le fait que ce VOC soit différent des métabolites naturels, il est possible d'abaisser le bruit de fond, ce dernier représentant le signal en l'absence du microorganisme. Cela a pour conséquence un abaissement de la limite de détection, et une limitation des risques de faux positifs.

Selon un autre objet, la présente invention concerne l'utilisation d'un procédé de l'invention pour détecter la présence de microorganisme(s) dans un milieu biologique, et de préférence dans un milieu physiologique.

L'utilisation d'un procédé de l'invention pour établir le phénotype d'un microorganisme, est décrite.

Selon encore un autre objet, la présente invention concerne l'utilisation d'un procédé de l'invention pour effectuer un test antibiogramme c'est-à-dire tester l'efficacité d'un antibiotique (AST : Antibiotic Susceptibility Testing). Il s'agit alors de détecter une croissance bactérienne en présence d'une concentration connue d'antibiotique.

Egalement décrit est l'utilisation d'un procédé de l'invention pour effectuer une numération de microorganismes par la méthode du nombre le plus probable (MPN : Most Probable Number). Il permet avantageusement de déterminer si, dans un volume élémentaire, il existe une bactérie. Pour la numération des bactéries, il s'agit de diviser le volume d'analyse en plusieurs sous-volumes et de détecter ou non la croissance bactérienne dans chacun des sous-volumes. Les résultats sont alors utilisés pour dénombrer un nombre de bactéries.

L'utilisation d'un procédé de l'invention pour tester, par le biais d'une caractérisation enzymatique, certaines résistances aux antibiotiques est décrite. Par exemple, une activité enzymatique VanX est caractéristique d'un haut niveau de résistance à la vancomycine. Elle peut être testée avec des substrats qui, une fois métabolisés, libéreront du thiophénol, ou de la 4-nitroaniline.

Par ailleurs, le procédé de l'invention peut être utilisé pour effectuer le dépistage de souches résistantes à la vancomycine, par mise en évidence de l'enzyme VanX.

<u>**Métabolite volatil VOC**</u>

Comme il ressort de ce qui précède, cette molécule volatile est issue de la dégradation enzymatique d'un substrat enzymatique spécifique au microorganisme à détecter selon l'invention. Ainsi, un métabolite volatil VOC considéré par l'invention peut constituer, au moins en partie, un substrat enzymatique spécifique du microorganisme à détecter.

Il est généré au sein même du microorganisme et traverse la paroi cellulaire pour se retrouver dans la phase liquide ou semi-solide.

Ce VOC est, dans le cadre de la présente invention, générable qu'à partir du substrat enzymatique spécifique requis selon l'invention.

A ce titre, il est distinct des VOC ou encore métabolites naturels volatils susceptibles d'être générés lors de la croissance microbienne.

Ainsi, il est avantageusement distinct du $CO_2$.

Dans le cadre de la présente invention, le métabolite volatil VOC considéré possède une constante de Henry, $H_{cc}$, la plus élevée possible, c'est-à-dire privilégiant un état gazeux par rapport à un état liquide ou semi-solide.

Plus précisément, la loi de Henry détermine, pour un composé volatil, le coefficient de partition à l'équilibre entre une solution aqueuse diluée et une phase gazeuse. Si $C_{i,L}$ est la concentration volumique du composé volatil i dans la phase aqueuse (en g/m$^3$ ou en mol/m$^3$) et $C_{i,G}$ sa concentration volumique dans la phase gaz (dans la même unité : g/m$^3$ ou mol/m$^3$), alors la constante de Henry est la grandeur sans dimension suivante :

$$H_{CC} = \frac{C_{i,G}}{C_{i,L}}$$

Cette loi exprime que, pour un composé volatil donné, à une température et une pression données, on retrouve à l'équilibre toujours le même coefficient de partition entre les deux phases. Il est important de noter qu'il existe de nombreuses manières d'exprimer cette même loi, suivant la définition que l'on donne de la constante de Henry.

Des métabolites VOC à forte constante de Henry $H_{cc}$ présentent des valeurs de $5.10^{-2}$ et plus, allant jusqu'environ $10^2$, alors que des valeurs moyennes se situent plutôt autour de $5.10^{-4}$ à $5.10^{-2}$ et les valeurs faibles autour de $10^{-5}$.

Comme précisé ci-dessus, la constante de Henry $H_{cc}$ est susceptible de varier en fonction de certains paramètres physiques de la phase liquide ou semi-solide parallèlement considérée, tels que la pression, la salinité, la température, etc.

Ainsi, dans le cas d'un milieu de culture liquide, il est à la portée de l'homme de l'art d'ajuster certains paramètres

physiques de ce milieu pour générer un maximum de composé gazeux à partir du soluté. Cet aspect est plus détaillé ci-après.

Alternativement, dans le cas d'un milieu de culture solide ou semi-solide, de type gélose, on pourra influer sur les propriétés de pression de la phase gazeuse ou de température en favorisant la croissance microbienne pendant un certain temps (3-5 h) pour accumuler dans la phase liquide le métabolite VOC spécifique cible puis en stoppant la croissance microbienne.

Ainsi, la volatilisation du métabolite est favorisée en augmentant la température et/ou en baissant la pression.

La concentration en métabolite VOC cible dans la phase liquide augmente alors jusqu'à atteindre un plateau. Une fois que ce plateau est atteint, ce qui limite la cinétique de détection, c'est le transfert du métabolite cible d'une phase à l'autre, et non plus la croissance microbienne.

Dans la variante de réalisation où la matrice nanoporeuse est directement plongée dans la phase liquide (qui est la phase où il y aura toujours le plus de métabolite VOC cible), cette approche peut ne pas être considérée.

Qui plus est, les interactions entre la solubilité en milieu aqueux et la tension de vapeur conditionnent la volatilisation d'un produit donné à partir de la solution aqueuse.

Par exemple, un métabolite volatil VOC soluble dans l'eau mais présentant une tension de vapeur élevée se volatilise rapidement.

De préférence, un métabolite volatil VOC soluble dans l'eau tel que requis selon l'invention présente, à T = 20 °C, une tension de vapeur comprise entre $10^{-4}$ et $10^2$ mbar, de préférence une tension de vapeur supérieure à $10^{-3}$ mbar.

Selon une variante avantageuse, ce métabolite peut posséder, de part lui-même, des propriétés optiques (absorbance, fluorescence, luminescence) permettant sa détection par transduction optique.

Dans le cas d'une détection en absorbance, la matrice nanoporeuse associée est transparente ou peu absorbante dans la zone de détection, et de préférence n'absorbe pas dans de larges bandes du spectre UV-visible. L'adsorption et l'accumulation d'un métabolite VOC absorbant dans les pores de la matrice va se traduire par une augmentation de l'absorbance.

Dans le cas d'une détection en fluorescence, la matrice nanoporeuse associée est dénuée de fluorescence intrinsèque ou possède une faible fluorescence intrinsèque. L'adsorption et l'accumulation d'un métabolite VOC fluorescent dans les pores de la matrice va se traduire par une augmentation de la fluorescence de la matrice.

Selon un mode de réalisation, un métabolite VOC convenant à l'invention peut être choisi parmi les dérivés phénoliques, tels que les dérivés nitrophénoliques, cyanophénoliques, cyanonitrophénoliques, acétylphénoliques, propionylphénoliques, les dérivés thiophénoliques, les dérivés naphtoliques, les dérivés aniliques, tels que les dérivés nitroaniliniques, ou les dérivés naphtylaminiques.

A titre illustratif et non limitatif des métabolites VOC convenant tout particulièrement à l'invention peuvent notamment être cités les thiophénol, nitronaphtol, p-nitroaniline ou 2-naphthylamine.

Selon une variante de réalisation avantageuse, le métabolite volatil VOC formé peut être un photoacide ou une photobase.

Un certain nombre de molécules possèdent en effet des propriétés acido-basiques qui diffèrent entre l'état fondamental et l'état excité (après absorption d'un photon). Ainsi, l'excitation peut déclencher un transfert de proton. Par exemple, un substituant OH sur un naphthol, peut être exalté de sorte que le pKa* de ce groupe à l'état excité est beaucoup plus petit que le pKa à l'état fondamental. On peut aussi observer de la fluorescence avec une espèce telle que le 2-naphthol par exemple, car le transfert de proton photoinduit permet sa déprotonation en une espèce fluorescente, en l'occurrence le 2-naphtholate. De même, un métabolite volatil VOC, tel que le paranitrophénol (pNP), absorbe dans le visible, notamment sous sa forme basique.

Bien entendu, la nature chimique du métabolite est étroitement liée au mode de détection retenu dans le cas où le changement de propriétés optiques constaté au niveau de la matrice est directement induit par sa présence.

Si on envisage une détection par absorbance, le VOC formé peut être plus particulièrement choisi parmi les molécules suivantes:

- le p-cyanophénol dont le coefficient d'extinction molaire est de $(2{,}1\pm0{,}2).10^4$ $M^{-1}.cm^{-1}$ à 245,8 nm pour la forme neutre et de $(2{,}6\pm0{,}2).10^4$ $M^{-1}.cm^{-1}$ pour la forme anionique ;
- le p-nitrophénol (pNP) dont le coefficient d'extinction molaire $\varepsilon$ est de $(1{,}8\pm0{,}1).10^4$ $M^{-1}.cm^{-1}$ pour la forme anionique à 400 nm et de 8300 $M^{-1}.cm^{-1}$ pour la forme neutre à 317 nm (l'isomère o-nitrophénol absorbe moins ($\varepsilon$=3.10$^3$ $M^{-1}.cm^{-1}$ à 414 nm)) ;
- le m-cyano-p-nitrophénol (mCNpNP) dont le coefficient d'extinction molaire e est de 1,7.10$^4$ $M^{-1}.cm^{-1}$ à 405 nm ;
- le 2,6-dichloro-4-nitrophénol (DCNP) ;
- le 2,6-dichloro-4-acetylphénol dont l'absorption maximale est à pH 5,4 et à 334,2 nm avec $\varepsilon$=2,1.10$^4$ $M^{-1}.cm^{-1}$ à 340 nm ; ou
- les dérivés naphtholiques tels que l'$\alpha$-naphthol ou le $\beta$-naphthol (1-naphtol ou 2-naphtol), l'$\alpha$-naphthylamine ou la $\beta$-naphthylamine (1-naphthylamine ou 2-naphthylamine).

Pour permettre une détection en absorbance, ces dérivés peuvent nécessiter la présence d'une molécule sonde au niveau de la matrice. Par exemple, la β-naphthylamine peut intéragir avec le diméthylaminocinnamaldéhyde de façon à former un composé coloré. Le β-naphthol peut interagir avec la molécule dont le nom d'usage est "Fast Blue BB", de façon à former un composé coloré. Ces dérivés naphtholiques peuvent également présenter des propriétés de fluorescence exploitables.

Peuvent également être considérés le thiophénol, le 2,6-dichloro-4-propionylphénol, le 2,6-difluoro-4-acétylphénol, le 2,6-dibromo-4-acétylphénol, et la 4-nitroaniline (p-nitroaniline).

Dans le cas des dérivés naphtholiques possédant une plus faible absorbance, il peut être intéressant de faire interagir ces dérivés au niveau de la matrice avec un réactif comme le Fast Blue BB ou du p-diméthylaminocinnamaldéhyde de manière à accroître leur absorbance. Le fait de pouvoir accomplir cette révélation directement au niveau d'une phase gazeuse est particulièrement avantageux compte-tenu du caractère toxique de ces réactifs vis-à-vis des microorganismes. La viabilité des microorganismes présents dans la phase liquide ou semi-solide n'est alors pas affectée.

Si on envisage une détection par absorbance, le métabolite volatil VOC formé peut être plus particulièrement choisi parmi les molécules qui suivent :

- le p-nitrophénol (4-nitrophénol) :

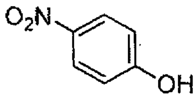

- le m-cyano-p-nitrophénol (3-cyano-4-nitrophénol) :

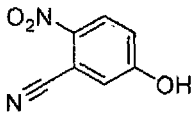

- le 2.6-dichloro-4-acétylphénol :

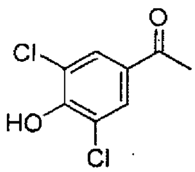

- le 2,6-dichloro-4-propionylphénol :

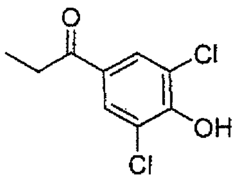

- le 2,6-difluoro-4-acétylphénol :

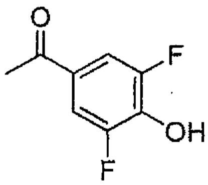

- le 2,6-dibromo-4-acétylphénol :

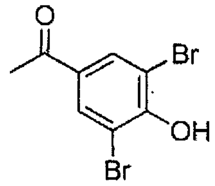

- la 4-nitroaniline (p-nitroaniline) :

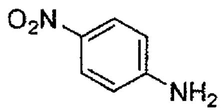

- la β-naphthylamine (2-naphthylamine) :

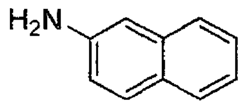

Si on envisage une détection par fluorescence, le métabolite volatil VOC formé peut être plus particulièrement choisi parmi les molécules qui suivent :

- l'ombelliférone (7-hydroxycoumarine) :

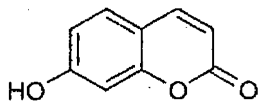

- la naphthazarine :

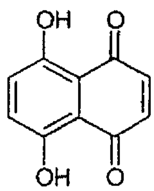

- la 4-trifluorométhylombelliférone (7-hydroxy-4-trifluorométhylcoumarine) :

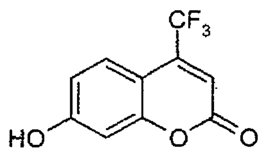

- la 4-méthylombelliférone (4-MU) :

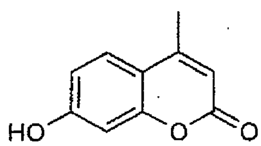

ainsi que parmi les photoacides et photobases suivants :

- le o-cyanophénol :

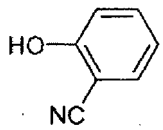

- le m-cyanophénol :

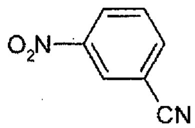

- le 1-naphtol :

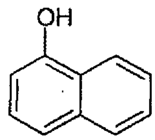

- le 2-naphtol :

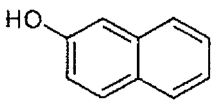

- le 6-cyano-2-naphtol :

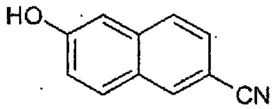

- le 6-hydroxyquinoline-N-oxyde :

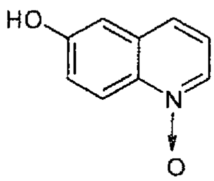

- le 2-méthyl-6-hydroxyquinoline-N-oxyde :

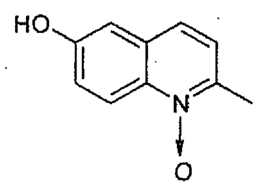

- la 6-hydroxyquinoline :

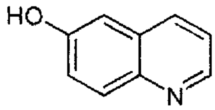

- la 7-hydroxyquinoline :

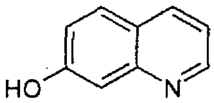

- la 8-hydroxyquinoline :

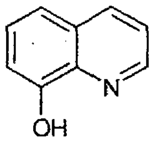

Selon un mode de réalisation préféré, le métabolite volatil VOC est choisi parmi le p-cyanophénol, le 4-nitrophénol (p-nitrophénol), le m-cyano-p-nitrophénol (3-cyano-4-nitrophénol), le 2,6-dichloro-4-nitrophénol, le 2,6-dichloro-4-acetylphénol, le thiophénol, le 2,6-dichloro-4-propionylphénol, le 2,6-difluoro-4-acétylphénol, le 2,6-dibromo-4-acétylphénol, 1'1-naphtol, le 2-naphtol, l'$\alpha$-naphthylamine, la $\beta$-naphthylamine, la 4-nitroaniline (p-nitroaniline), l'ombelliférone, la naphthazarine, la 4-trifluorométhylombelliférone, la 4-méthylombelliférone, le o-cyanophénol, le m-cyanophénol, le 6-cyano-2-naphtol, le 6-hydroxyquinoline-N-oxyde, le 2-méthyl-6-hydroxyquinoline-N-oxyde, la 6-hydroxyquinoline, la 7-hydroxyquinoline, ou la 8-hydroxyquinoline.

De préférence, pour une détection par fluorescence, le métabolite volatil VOC formé est choisi parmi l'ombelliférone, la naphthazarine, la 4-trifluorométhylombelliférone, la 4-méthylombelliférone (4-MU), un naphthol tel que le 2-naphthol ou un naphthylamine tel que la $\beta$-naphthylamine.

**Substrat enzymatique**

Comme il ressort de ce qui précède, un substrat enzymatique convenant à l'invention figure plus particulièrement un composé non volatil apte, d'une part, à être métabolisé spécifiquement par le microorganisme recherché, et, d'autre part, à libérer, précisément lors de sa métabolisation, au moins une molécule d'un métabolite volatil conforme à l'invention, c'est à dire tel que défini ci-dessus.

Avantageusement, un substrat enzymatique de l'invention peut être de nature phénolique ou naphtolique, au regard du métabolite VOC considéré, à savoir dérivé du phénol ou du naphtol.

Pour des raisons évidentes, le choix de ce substrat s'effectue en fonction de la voie enzymatique que l'on cherche à caractériser et donc de l'enzyme ciblée chez le ou les microorganisme(s) que l'on veut détecter, telle que par exemple une voie impliquant des glucuronidases, des glucosidases, des estérases, des lipases, des phosphatases alcalines, etc.

Par exemple, une activité $\beta$-D-glucuronidase est caractéristique de la présence de coliformes, par exemple *d'Escherichia coli* et une activité L-alanine aminopeptidase permet de différencier les bactéries Gram positives des bactéries Gram négatives.

A l'inverse, l'analyse peut se fonder sur la caractérisation d'un défaut d'activité enzymatique. Par exemple, l'absence d'activité $\beta$-galactosidase, est caractéristique des salmonelles, et l'absence d'activité peptidase sur un substrat tel que le D-alanine-p-nitroaniline est caractéristique de *Listeria monocytogenes* (les autres *Listeria* possédant cette activité enzymatique).

Ainsi le substrat considéré selon l'invention peut dériver des substrats glycosidases, tels que les $\alpha$-galactosidase, $\beta$-galactosidase, $\alpha$-glucosidase, $\beta$-glucosidase, cellobiosidase, $\beta$-glucuronidase, $\beta$-ribofuranosidase, hexosaminidase, $\alpha$-mannosidase, $\beta$-mannosidase, $\beta$-xylosidase, naringinase, arabinosidase, amylase, $\alpha$-maltosidase et $\beta$-maltosidase, des substrats estérases, tels que les lipase, phosphatase et phosphatase alcaline, des substrats peptidases, tels que les arylamidase, trypsine, dipeptidase et carboxypeptidase, des substrats deoxyribonucléases, des $\beta$-lactamase et des nitroréductases.

Ce choix de substrat peut également être ajusté au regard de la nature chimique du métabolite retenu à des fins de détection.

En effet, comme il ressort de ce qui précède, et selon une variante préférée, le substrat enzymatique considéré doit se prêter à une métabolisation pour libérer, via la rupture du lien covalent figurée par la fonction précisément métabolisable (ester, éther, amide...), au moins une molécule dudit métabolite VOC.

Il est entendu que la nature de la fonction métabolisable, à savoir ester, éther ou encore peptide, phosphoester, phosphodiester ou amide, et plus précisément sa création dans le substrat, peut imposer le choix d'un métabolite VOC particulier. Il est ainsi nécessaire que ce métabolite se prête à un couplage covalent de ce type.

Par exemple, le p-nitrophénol, considéré comme un métabolite volatil VOC selon l'invention, se prête, du fait de son hydroxyle -OH, à un couplage avec des sucres (substrat glucuronidase par exemple), des acides gras (substrat estérase ou lipase) ou un acide phoshorique (substrat phosphatase).

En revanche, un tel métabolite ne se prête pas à un couplage avec des substrats peptidase. Il faut alors envisager un métabolite volatil VOC présentant une amine $-NH_2$, comme la p-nitroaniline ou la 2-naphthylamine ou présentant un thiophénol, comme par exemple les substrats peptidases qui libèrent de l'α-phénylthioglycine.

La sélection de ce métabolite VOC et du type de fonction métabolisable dédiées à former le substrat requis selon l'invention relève clairement des compétences de l'homme de l'art.

Par exemple, des substrats peptidases libèrent de l'α-phénylthioglycine. Celle-ci se décompose spontanément en ammoniac et thiophénol. Le thiophénol est alors le métabolite volatil cible, qui peut être détecté, en absorbance, avec une sonde, le 5,5'-dithio-bis-(2-nitrobenzoic acid), connu sous le nom de réactif d'Ellman, comme décrit sur le schéma suivant :

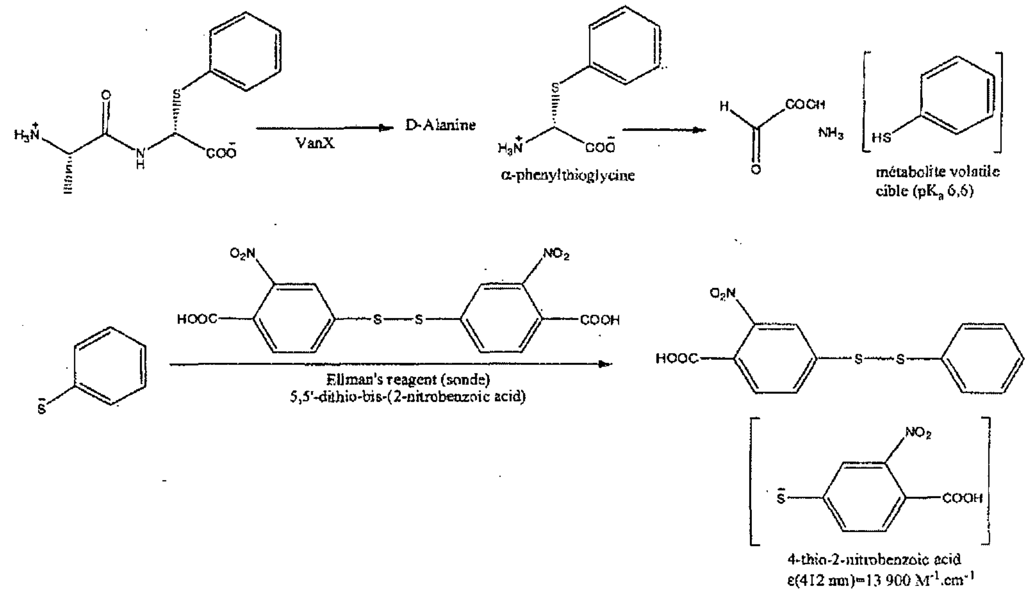


La détection de thiophénol grâce à la matrice nanoporeuse dopée avec le réactif d'Ellman revient à mettre en évidence la résistance à la vancomycine.

A titre d'exemples non exhaustifs de substrat enzymatique convenant à l'invention, apte à libérer un métabolite VOC, on peut citer les composés suivants :

- les substrats d'estérases tels que par exemple :

- le 4-nitrophényl octanoate (CAS 1956-10-1)

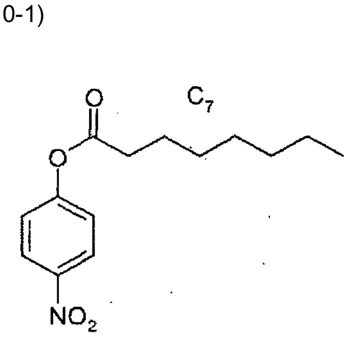

- le 4-nitrophényl myristate (CAS 146717-85-7)

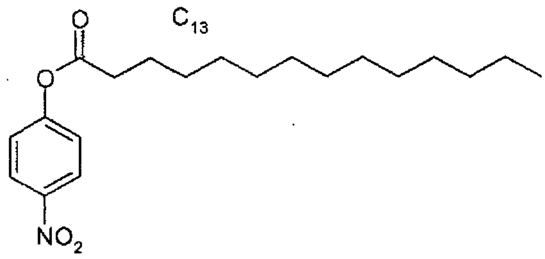

- le 4-nitrophényl dodécanoate (CAS 1956-11-2)

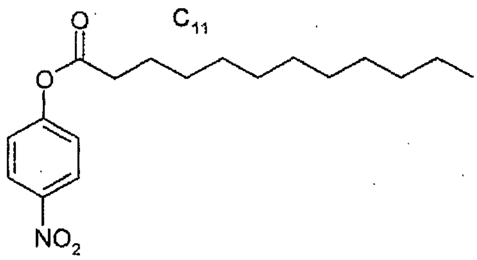

- les substrats de β-glucuronidases, tels que par exemple le 4-nitrophényl β-D-glucuronide (CAS 10344-94-2) :

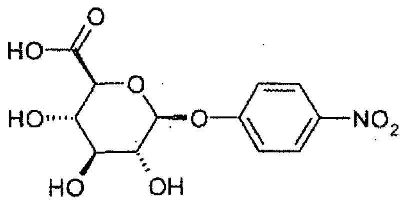

- les substrats de β-galactosidases, tels que par exemple le 4-nitrophényl β-D-galactopyranoside (CAS 3150-24-1) :

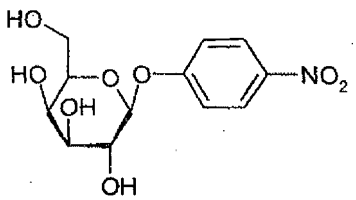

ou le composé de formule suivante :

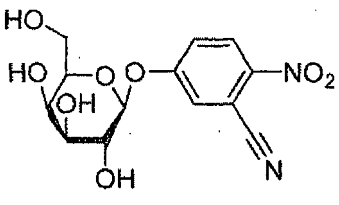

- les substrats de β-glucosidases, tels que par exemple que le 4-nitrophényl β-D-glucopyranoside (CAS 2492-87-7) :

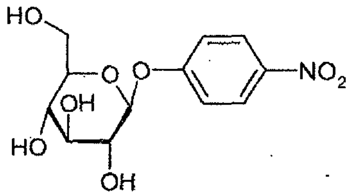

- les substrats d'α-galactosidases, tels que par exemple le 4-nitrophényl α-D-galactopyranoside (CAS 7493-95-0) :

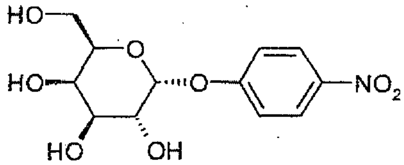

- les substrats d'α-glucosidases, tels que par exemple le 4-nitrophényl α-D-glucopyranoside (CAS 3767-28-0) :

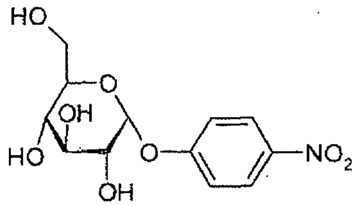

ou le riaphtalene α-D-glucopyranoside :

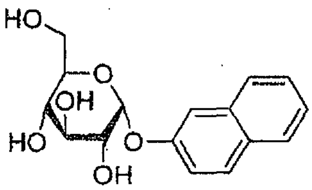

- les substrats d'α-mannosidases, tels que par exemple le 4-nitrophényl α-D-mannopyranoside (CAS 10357-27-4) :

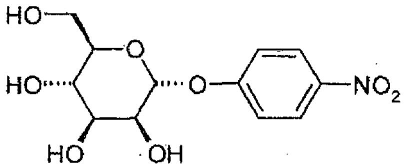

- les substrats de β-mannosidases, tels que par exemple le 4-nitrophényl β-D-mannopyranoside (CAS 35599-02-1) :

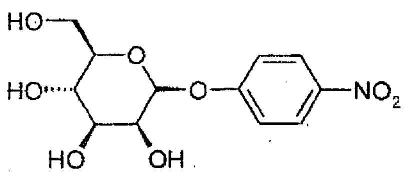

- les substrats de β-xylosidases, tels que par exemple le 4-nitrophényl β-D-xylopyranoside (CAS 2001-96-9) :

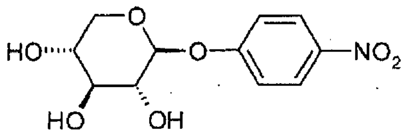

- les substrats de β-xylosidases, tels que par exemple le 4-nitrophényl α-L-arabinofuranoside (CAS 6892-58-6) :

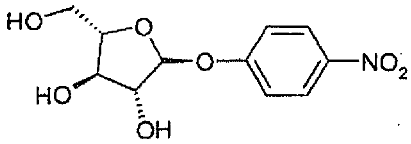

- les substrats de naringinases, tels que par exemple le 4-nitrophényl α-L-rhamnopyranoside (CAS 18918-31-5) :

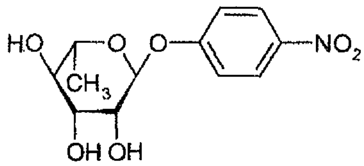

- les substrats d'arabinosidases, tels que par exemple le 4-nitrophényl α-L-arabinopyranoside (CAS 1223-07-0) :

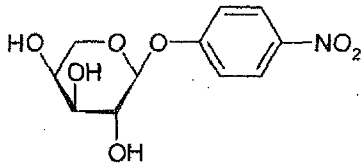

- les substrats d'amylases, tels que par exemple le 4-nitrophényl α-L-arabinopyranoside (CAS 66068-38-0) :

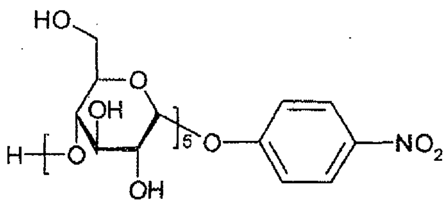

ou le composé de formule suivante :

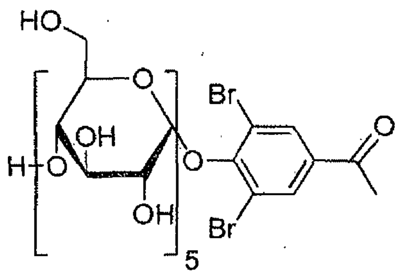

- les substrats d'alkalines phosphatases, tels que par exemple le 4-nitrophényl phosphate disodium salt (CAS 4264-83-9) :

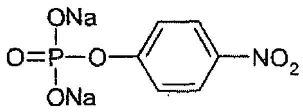

ou le composé de formule suivante :

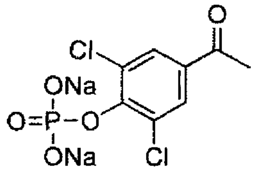

ou le composé de formule suivante :

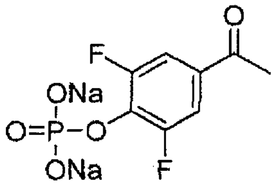

- les substrats d'α-maltosidases, tels que par exemple le 4-nitrophényl α-D-maltopyranoside (CAS 17400-77-0) :

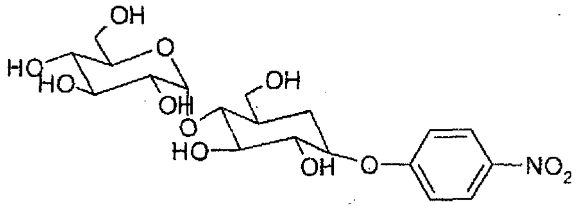

- les substrats de β-maltosidases, tels que par exemple le 4-nitrophényl β-D-maltopyranoside (CAS 56846-39-0) :

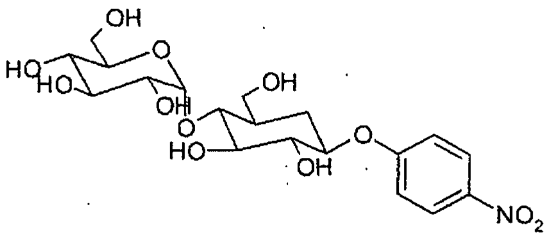

ou le composé de formule suivante :

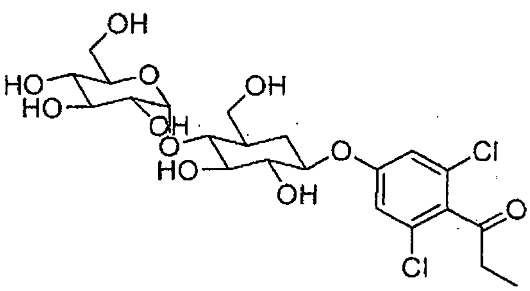

- les substrats de N-acétyl-β-glucosaminidase, tels que par exemple les composés de formule suivante :

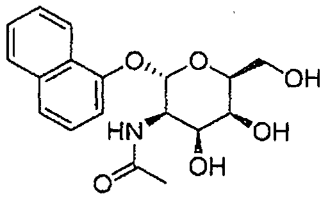

- les substrats de peptidase, tels que par exemple les composés de formule suivante :

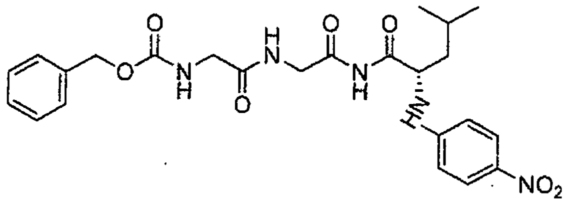

- les substrats de trypsine, tels que par exemple les composés de formule suivante :

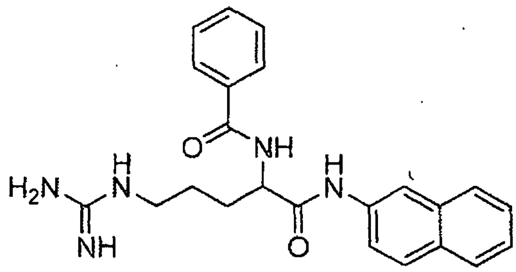

De préférence, les substrats enzymatiques dédiés à former le substrat requis sont choisis parmi les substrats de sucrases tels que les glucosidases, les glucuronidases ou les glactosidases, les substrats peptidases, tels que les arylamidases ou les dipeptidases comme les substrats VanX et les substrats estérases tels que les lipases et les phosphatases.

Comme précisé ci-dessus ce substrat considéré en étape 1) du procédé selon l'invention est mis en oeuvre dans une phase liquide ou semi-solide susceptible de contenir le ou les microorganismes recherchés.

Selon une variante de réalisation, l'étape 2) d'un procédé de l'invention peut mettre en oeuvre plusieurs substrats enzymatiques distincts, respectivement métabolisables par des microorganismes distincts. Lorsqu'un procédé de l'invention met en oeuvre plusieurs substrats enzymatiques distincts, ceux-ci génèrent de préférence lors de leur métabolisation des métabolites VOC distincts. Ce mode de réalisation est utilisé généralement pour identifier un ou plusieurs microorganismes. Alternativement, lorsqu'un procédé de l'invention met en oeuvre plusieurs substrats enzymatiques distincts, ceux-ci peuvent générer le même métabolite VOC. Cette alternative est alors utilisée à des fins de détection d'un ou plusieurs microorganismes.

Selon un autre mode de réalisation, un procédé de l'invention peut mettre en oeuvre à l'étape 2) plusieurs substrats enzymatiques générant des métabolites VOC distincts et métabolisables par un même microorganisme.

**<u>Phase liquide ou semi-solide</u>**

La phase liquide ou semi-solide considérée selon l'invention peut être formée en tout ou partie du milieu biologique susceptible de contenir au moins une forme vivante dudit microorganisme, des éléments nutritifs nécessaires à la prolifération dudit microorganisme, et d'un substrat enzymatique, tel que défini ci-dessus, métabolisable par ledit microorganisme en un métabolite VOC conforme à l'invention.

Une phase semi-solide selon l'invention s'entend notamment d'une phase liquide structurée, par exemple sous la forme d'un gel. A titre d'exemple de phase semi-solide convenant à l'invention, on peut citer les géloses habituellement utilisées pour la culture de microorganismes, telles que les bactéries.

Le choix des éléments nutritifs nécessaires à la prolifération du microorganisme relève clairement des compétences de l'homme de l'art.

On peut citer comme éléments nutritifs l'eau, les protéines, les lipides, les glucides, les vitamines, les éléments

minéraux (C, H, O, N, P, Ca, Mg, K, Na, S, etc.), les oligo-éléments (I, Cu, Co, Cr, Mn, V, Mo, Ni, Pb, Cl, Fe, B, etc.), les acides aminés essentiels, les acides essentiels et/ou les fibres alimentaires (cellulose, hémicellulose, etc.).

L'ajout de ces éléments nutritifs relève clairement des compétences de l'homme de l'art.

Qui plus est, de nombreux milieux nutritifs spécifiques ou non à la prolifération de certains microorganismes sont disponibles commercialement.

La sélection du milieu adéquat et la mise en présence de celui-ci avec le milieu biologique à analyser, à des fins de constituer la phase liquide ou semi-solide devant en outre être complémentée en substrat enzymatique métabolisable, relèvent d'opérations de routine pour l'homme du métier.

Comme expliqué précédemment, la loi de Henry régit la répartition du métabolite volatil VOC entre la phase liquide ou semi-solide et la phase gaz. Il est donc avantageux d'ajuster, si possible, les propriétés de la phase liquide ou semi-solide afin de privilégier une constante de Henry la plus élevée possible pour le métabolite VOC considéré.

Or, comme précisé précédemment, la constante de Henry dépend, notamment, du pH, de la température de la phase liquide ou semi-solide et de la pression de la phase gazeuse au-dessus des phases liquides ou semi-solides.

Ainsi, un aspect de mise en oeuvre d'un procédé de l'invention pourra consister à ce que le pH de la phase liquide ou semi-solide soit ajusté pour privilégier la forme moléculaire du VOC à l'une de ses formes ioniques.

Il est particulièrement pertinent d'influer sur le pH du milieu liquide ou semi-solide pour les composés protiques qui se dissocient sensiblement dans l'eau tels que les acides carboxyliques, les alcools, les phénols, les thiols (aromatiques ou aliphatiques), les amines, les anilines et les pyridines. Seule l'espèce moléculaire non dissociée est susceptible de se volatiliser.

Ainsi, selon un mode de réalisation, le pH de la phase liquide ou semi-solide est ajusté pour privilégier la forme moléculaire du VOC à l'une de ses formes ioniques.

Par exemple, dans le cas de l'émission du p-nitrophénol pNP, le pH de la phase liquide ou semi-solide sera avantageusement stabilisé à une valeur sensiblement inférieure au $pK_a$ (7,15), de manière à ce que la forme moléculaire prédomine (en tamponnant à 6,1 avec du MES, l'acide 4-morpholinoéthanesulfonique, par exemple).

De même, la constante de Henry est plus élevée lorsque l'on augmente la température. Cependant, une élévation de température n'est généralement pas compatible avec les exigences des cultures microbiennes qui requièrent généralement une température d'incubation entre 35 et 37 °C. Pour une température supérieure, il est généralement constaté une baisse de l'activité microbienne (stress thermique).

La constante de Henry dépend également d'autres paramètres notamment le transfert de matière, la présence de tensioactifs, la présence d'un solide en suspension, la présence d'un cosolvant, la salinité. Il est donc possible d'ajuster ces paramètres afin d'obtenir une constante de Henry élevée.

La présence d'un tensioactif peut être préjudiciable à l'émission du métabolite volatil VOC en phase gaz. Il peut donc être souhaitable de s'affranchir dans les milieux de culture considérés, d'inhibiteurs de type tensioactif (désoxycholate de sodium par exemple).

Ainsi, selon un mode de réalisation préférée la phase liquide ou semi-solide pourra comprendre une quantité réduite, voire nulle en tensio-actif.

La présence d'un solide divisé en suspension (colloïde) peut également se révéler préjudiciable pour la volatilisation d'un métabolite volatil VOC s'il s'adsorbe sur les particules, ce qui pourra être notamment le cas pour des métabolites volatils VOC hydrophobes. C'est un phénomène à prendre en considération car les milieux pour l'hémoculture contiennent souvent des colloïdes (charbon actif ou résine) dont le rôle principal est d'adsorber les antibiotiques pour les rendre inactifs.

Tout liquide miscible avec l'eau, comme un alcool par exemple, présent dans la phase aqueuse en quantité sensible est un cosolvant. Si la présence de ce dernier accroît la solubilité du métabolite volatil VOC dans la phase aqueuse, alors il est préférable que le milieu de culture en contienne en quantité réduite voir nulle.

Les solubilités dans l'eau de différents types de composés (protéines, métabolites volatils VOC, tensioactifs, gaz, etc.) sont également susceptibles d'être affectées par la présence de sels inorganiques. Ce phénomène, appelé relargage (*salting-out effect),* a un impact favorable sur la constante de Henry : un accroissement de la salinité de la phase aqueuse s'accompagne d'une hausse de la constante. En conséquence, sous réserve que la présence d'un tel composé ne soit pas par ailleurs préjudiciable au développement du microorganisme que l'on cherche à caractériser, il peut être avantageux de considérer ce type de composé. Néanmoins, il est également requis que les milieux de culture soient en général isotoniques ou presque, soit l'équivalent de 158 mM NaCl, de manière à ne pas induire un stress osmotique chez les microorganismes qu'ils contiennent.

Selon une variante de réalisation, un procédé selon l'invention est caractérisé en ce que la matrice est disposée en phase gazeuse et en ce que l'étape 2) peut mettre en oeuvre des moyens optimisant la circulation dudit métabolite volatil VOC vers la phase gazeuse. Ces moyens peuvent être figurés par une agitation mécanique ou une nébulisation en surface de la phase liquide ou semi-solide.

Ainsi, pour favoriser le transfert de matière (le métabolite volatil VOC) vers la phase gazeuse, on pourra recourir à une agitation mécanique de la phase liquide par barreau aimanté, pâle et contre-pâle. Pour ce qui est de la nébulisation,

elle vise à créer un brouillard à l'interface des deux phases, de manière à maximiser leur surface de contact.

Particulièrement, dans le cas d'une phase liquide, ces moyens optimisants peuvent être figurés par un ruissellement de la phase liquide sur une phase solide inerte divisée, telle qu'une accumulation de billes de verre, afin d'accroître la surface de l'interface liquide-gaz.

En conséquence, et au regard de ce qui précède, il peut être avantageux de privilégier dans un procédé selon l'invention d'un milieu avec une salinité élevée dans lequel on favorisera le transfert de matière grâce à une méthode d'agitation ou à une méthode de nébulisation, et dans lequel on évitera la présence d'inhibiteurs de type tensioactif, d'un solide divisé en suspension et d'un cosolvant accroissant la solubilité du métabolite volatil VOC.

## Matrice nanoporeuse

Comme il ressort de ce qui précède, une matrice considérée selon l'invention est destinée à interagir avec le métabolite recherché si présent. En d'autres termes, elle possède une affinité à l'égard du métabolite VOC considéré.

Cette affinité peut notamment être conditionnée *via* un ajustement des propriétés de la matrice nanoporeuse qui seront sélectionnées pour être adaptées aux propriétés du métabolite volatil VOC émis, notamment au niveau de son pH et/ou de la taille de ses pores, afin d'assurer une affinité et une capture optimale.

Par ailleurs, avantageusement, la matrice nanoporeuse peut présenter une surface spécifique de 300 à 1000 $m^2.g^{-1}$, voire de 300 à 900 $m^2.g^{-1}$, et en particulier, de 400 à 900 $m^2.g^{-1}$.

Plus particulièrement, une matrice nanoporeuse telle que requise selon l'invention est de préférence transparente, sans fluorescence intrinsèque, inerte vis-à-vis d'une sonde et développe une grande surface massique.

Par exemple, pour une matrice nanoporeuse dédiée à capter le p-nitrophénol (pNP), des conditions basiques sont privilégiées pour permettre la déprotonation du pNP après capture dans la matrice. Ainsi, un coefficient d'extinction molaire maximum $\varepsilon_{max}$ de 18100 $M^{-1}.cm^{-1}$ est obtenu pour le phénolate $pNP^-$, alors qu'il n'est que de 8300 $M^{-1}.cm^{-1}$ pour le phénol pNP.

De même, les propriétés fluorescentes d'un métabolite VOC comme la 4-méthylombelliférone dépendent du pH environnant (ce qui en fait d'ailleurs une sonde de pH en fluorescence). Il est ainsi nécessaire de maintenir la 4-méthylombelliférone adsorbée dans des conditions de protonation qui soient stables.

Une matrice de l'invention sera préférentiellement constituée d'un matériau solide, organique ou inorganique, dédié au piégeage du métabolite VOC formé et révélateur de la présence du microorganisme recherché.

De préférence, ladite matrice est constituée d'un matériau organique, inorganique ou hybride organique-inorganique.

Elle est avantageusement formé en tout ou partie d'un matériau inorganique ou hybride organique-inorganique poreux.

Les pores seront les plus petits possibles, pour accroître la surface spécifique et augmenter la sélectivité du détecteur, mais suffisamment grands, pour permettre le passage du métabolite volatil VOC dans les nanopores.

Préférentiellement, une matrice nanoporeuse convenant à l'invention peut posséder une distribution de taille de pores ajustée à la taille dudit métabolite VOC, et en particulier inférieure à 100 nm, et de préférence variant de 3 à 100 Å.

Dans ces conditions, même lorsque la matrice est placée dans le milieu nutritif, les pores forment un filtre, laissant passer les métabolites volatils, et de préférence, uniquement ces derniers. Ainsi, la matrice peut être placée dans le milieu nutritif, ou dans un gaz, à proximité du milieu nutritif

Dans une première forme de mise en oeuvre, une matrice organique ou hybride organique-inorganique convenant à un procédé de l'invention peut être obtenue à partir d'un matériau polymère.

Par « matériau polymère », on entend, dans le cadre de la présente invention, un (co)polymère naturel ou synthétique, soluble ou insoluble et notamment organique, ledit matériau étant avantageusement nanoporeux.

Le matériau polymère utilisé dans la présente invention peut être un hydrogel. Ainsi, le matériau polymère utilisable dans le cadre de la présente invention peut être choisi parmi l'agarose ; la gélatine ; la cellulose ; la carboxymethylcellulose ; un alginate ; une polyoléfine ; un polymère styrènique comme une résine polystyrène avantageusement poreuse ; un polymère hydrocarboné halogéné comme du polytetrafluoroéthylène ou du poly(chlorotrifluoroéthyléne); un polymère vinylique comme un poly(vinyl décanoate) ou du polyalcool de vinyle ; un polymère (méth)acrylique comme un poly(n-butyl acétate) ou un poly(benzyl méthacrylate) ; du polyéthylène glycol ; du poly(propylène fumarate) ; du poly(éthylène fumarate) ; un poly(alpha-hydroxyester) ; un poly(orthoester) ; un polyanhydride ; un poly(phosphazene) ; un poly(ester amide) ; un acide polylactique ; un acide polyglycolique ; le polycaprolacton (PCL) ; le polydioxanone (PDO) ; un polyuréthane ; un gel de cholesteryl anthraquinone-2-carboxylate et de polyméthylsiloxane ; un gel de 1,3:2,4-dibenzylidenesorbitol et d'octamethylcyclotetrasiloxane ; un gel de diamide aromatique à chaine perfluorée et de perfluorotributylamine notamment décrit dans l'article de Loiseau et al., 2002, Tetrahedron, vol. 58, pages 4049-4052.

Il peut également s'agir d'un polymère à microporosité intrinsèque (PIM: polymer of intrinsic microporosity), tel que des polydioxanes ou des polyimides, ou un matériau silicone, tel que des polysiloxanes.

Dans une seconde forme de mise en oeuvre, une matrice nanoporeuse de l'invention peut être une matrice sol-gel nanoporeuse d'oxydes métalliques et plus particulièrement d'un oxyde métallique hybride organique-inorganique, et sa distribution de tailles de pores sera avantageusement adaptée à la taille du composé volatil cible.

Par « matrice sol-gel nanoporeuse d'oxydes métalliques », on entend un réseau polymérique nanoporeux élaboré à partir d'au moins un oxyde métallique de formule (IV) :

$$M(X)_m(OR_a)_n(R_b)_p$$

dans laquelle :

- M correspond à un métal choisi parmi le silicium, l'aluminium, le tungstène, le titane, le zirconium, le niobium, le vanadium, le tantale, le yttrium et le cérium,
- $R_a$ correspond à un radical alkyle en $C_1$ à $C_6$ ou à un aryle en $C_5$,
- $R_b$ correspond à un radical alkyle en $C_1$ à $C_6$, à un aryle en $C_5$ à $C_{10}$ ou à un aminoalkyle en $C_3$ à $C_6$,
- n, m et p sont des entiers, tels que leur somme est égale à la valence de M et n est supérieur ou égal à 2, m et p pouvant être égaux à 0, et
- X est un halogène, de préférence le chlore.

Selon un mode préféré de réalisation, le métal M de l'oxyde précurseur de la matrice sol-gel est du silicium ou de l'aluminium ou du zirconium. Selon un mode particulièrement préféré de réalisation, l'oxyde métallique est $Si(OMe)_4$.

De préférence, ladite matrice dérive de la polycondensation d'alkoxysilane.

Alternativement, une matrice de l'invention peut dériver de la polycondensation d'alcoolate de formule $M(OR)_n$ et R' $M(OR)_{n-1}$ avec M étant choisi parmi Si, Al, W, Ti, Nb, Zr, Ta, et V.

De préférence, une matrice nanoporeuse de l'invention peut dériver de la polycondensation d'un premier polyalcoxysilane qui est le tétraméthoxysilane ($Si(OMe)_4$) et d'un ou plusieurs second(s) polyalcoxysilane(s) différent(s) du premier polyalcoxysilane, dans un rapport molaire $Si(OMe)_4$/second(s) polyalcoxysilane(s) de 1/0,01 à 1/1, de préférence de 1/0,01 à 1/0,50, notamment de 1/0,01 à 1/0,30, particulièrement de 1/0,01 à 1/0,15, tout particulièrement de 1/0,02 à 1/0,06

Le matériau sol-gel de l'invention est poreux et il a une distribution de taille de pores allant de 3 à 100 angströms et une surface spécifique de 300 à 1000 $m^2.g^{-1}$.

Les seconds polyalcoxysilanes entrant dans la composition du matériau revendiqué peuvent être n'importe lesquels des polyalcoxysilanes connus dans la littérature, notamment les suivants : (N-(3-(triméthoxysilyl)propyl)éthylènediamine ; 3-aminopropyltriéthoxysilane ; 3-aminopropyltriméthoxysilane ; (3-(méthylamino)propyl)triméthoxysilane ; 1-(3-(triméthoxysilyl)propyl)urée ; N-(3-(triméthoxysilyl)propyl)aniline ; N-1-(3-(triméthoxysilyl)propyl)diéthylènetriamine ; bis(3-(méthylamino)propyl)triméthoxysilane ; diéthoxydiéthylsilane ; diéthoxydiméthylsilane ; diéthoxy(méthyl)vinylsilane ; 1,3-diéthoxy-1,1,3,3-tetraméthyldisiloxane ; diméthoxyméthylvinylsilane ; méthyldiéthoxysilaaie ; chloro-méthoxy-diméthylsilane ; éthoxy(diméthyl)vinylsilane ; éthoxytriméthylsilane ; méthoxytriméthylsilane ; diéthoxydiéthylsilane ; diéthoxydiméthylsilane ; diéthoxy(méthyl)vinylsilane ; 1,3-diéthoXy-1,1,3,3 -tetraméthyl disiloxane ; diméthoxydiméthylsilane ; diméthoxyméthylvinylsilane ; méthyldiéthoxysilane ; 1,2-bis(triéthoxysilyl)éthane ; 1,2-bis(triméthoxysilyl)éthane ; (chlorométhyl)triéthoxysilane ; 1,3-diméthyltetraméthoxydisiloxane ; éthyltriméthoxysilane ; triéthoxy(éthyl)silane ; triéthoxyméthylsilane ; triéthoxyméthylsilane ; triéthoxy(vinyl)silane ; triméthoxyméthylsilane ; triméthoxyméthylsilane ; triméthoxy(vinyl)silane ; triméthoxy(vinyl)silane. ; tétraéthoxysilane .

On peut citer en particulier les suivants : 3-aminopropyltriéthoxysilane, 3-aminopropyltriméthoxysilane et plus particulièrement ce dernier.

Des matériaux sol-gel particulièrement préférés sont préparés essentiellement à partir de tétraméthoxysilane et de 3-aminopropyltriéthoxysilane (APTES) dans un rapport molaire $Si(OMe)_4$/APTES de 1/0,01 à 1/0,30, de préférence de 1/0,01 à 1/0,15, notamment de 1/0,02 à 1/0,06, tout particulièrement de 1/0,03.

Pour rappel, un matériau sol-gel est un matériau obtenu par un procédé sol-gel consistant à utiliser comme précurseurs des alcoolates de formule M(OR)n où M est un métal, notamment le silicium, et R un groupement alkyle, et à les hydrolyser. En présence d'eau, l'hydrolyse des groupements alkoxy (OR) intervient, formant des petites particules de taille généralement inférieure à 1 nanomètre. Ces particules s'agrègent et forment des amas qui restent en suspension sans précipiter, et forment un sol. L'augmentation des amas augmente la viscosité du milieu qui gélifie. Un matériau sol-gel est obtenu par séchage du gel, en évacuant le solvant en dehors du réseau polymérique formé.

Le matériau sol-gel de l'invention est essentiellement préparé à partir de 2 à 4 polyalcoxysilanes, notamment de 2 ou 3 et particulièrement de 2 polyalcoxysilanes. Le matériau final peut contenir de 50 à 95% de dérivés de polyalcoxysilanes.

Des catalyseurs des réactions d'hydrolyse et/ou de polycondensation des polyalcoxysilanes peuvent être ajoutés au sol de départ. Ceux-ci peuvent être un acide organique ou inorganique (acide chlorhydrique, acide sulfurique, acide nitrique, acide acétique, acide tartrique, acide phtalique, acide maléique, etc.), une base organique ou inorganique (amie primaire, ammoniaque, soude, 4-amino 3-benzène 2-one, etc.) ou un anhydride d'acide (acide chlorhydrique gazeux, anhydride maléique, anhydride succinique, etc.).

C'est pourquoi un matériau sol-gel de l'invention peut en outre renfermer un catalyseur des réactions d'hydrolyse des polyalcoxysilanes ou un catalyseur de polycondensation des polyalcoxysilanes, ou les deux. Ces catalyseurs restent dans le matériau final dans des proportions pouvant aller de 1 à 50%.

Des composés structurants (polymères organiques, surfactants neutres, surfactants anioniques, surfactants cationiques; etc.), permettant d'obtenir une structure poreuse régulière et/ou des formes de cavité particulières, peuvent aussi être ajoutés à condition qu'ils puissent être éliminés par lavage ou calcination sans détériorer les propriétés optiques et structurales de la matrice.

Le procédé de préparation d'une matrice sol-gel nanoporeuse d'oxydes métalliques est décrit plus en détail dans la demande WO 2007/031657.

Dans une variante de réalisation, une matrice nanoporeuse de l'invention peut comprendre au moins une molécule sonde, apte à amplifier le signal de transduction optique via son interaction avec ledit métabolite VOC piégé dans ladite matrice. Une telle sonde peut être une molécule capable de réagir rapidement et totalement avec le métabolite volatil VOC de manière à former un composé plus absorbant ou plus fluorescent.

Par « molécule sonde », on désigne toute molécule organique portant une fonction réactive dont la réaction avec un métabolite VOC entraîne une modification d'au moins une de ses propriétés physico-chimiques détectable par une technique d'analyse appropriée, préférentiellement une modification de ses propriétés spectrales détectable par spectrophotométrie.

La sélection d'une molécule sonde convenant à un procédé selon l'invention relève clairement des compétences de l'homme de l'art.

De telles molécules sondes sont notamment décrites dans WO 2007/031657, dont le contenu est incorporé à titre de référence. Comme molécule sonde convenant à un procédé de l'invention, on peut notamment citer le chlorure de 4-benzoylamino-2,5-diéthoxybenzènediazonium (Fast Blue BB) pour la détection en absorbance du naphthol, le diméthylaminocinnamaldéhyde (DMACA) pour la détection en absorbance de la naphthylamine et le 5,5'-dithio-bis-(2-nitrobenzoic acid), dit réactif de Ellman, pour la détection en absorbance du thiophénol.

Avantageusement, l'étape d'élaboration de la matrice sol-gel et celle d'incorporation d'au moins une molécule sonde, si présente, seront réalisées simultanément. En effet, les conditions de préparation sont généralement suffisamment douces pour que les molécules sondes soient incorporées dans la matrice sol-gel sans être altérées.

Selon un autre mode préféré de réalisation, l'incorporation d'au moins une molécule sonde pourra se faire dans la matrice nanoporeuse également par imprégnation en solution ou en phase vapeur selon des techniques bien connues de l'homme du métier, dont notamment la sublimation.

Différents modes d'agencement, plus précisément décrits ci-après, peuvent être considérés pour la matrice nanoporeuse, avec dans tous les cas sa localisation obligatoire dans une phase liquide ou dans une phase gazeuse contigüe à la phase liquide ou semi-solide contenant le microorganisme à caractériser.

**<u>Modes de détection optique des métabolites volatils VOC</u>**

Comme il ressort de ce qui précède, c'est l'adsorption et l'accumulation du métabolite volatil VOC au niveau des pores de la matrice qui va induire la modification des propriétés optiques de la matrice nanoporeuse et permettre de révéler la présence du microorganisme correspondant.

Selon un premier mode de réalisation, le mode de détection du métabolite VOC est la détection en absorbance. La matrice nanoporeuse associée est alors de préférence transparente ou peu absorbante dans la zone de détection. Une telle matrice n'absorbe pas dans de larges bandes du spectre UV-visible. L'adsorption et l'accumulation d'un métabolite volatil VOC absorbé dans les pores de la matrice, va se traduire par une augmentation de l'absorbance.

Dans un mode de détection en absorbance, la matrice peut adopter avantageusement une géométrie de guide d'onde. Cela permet d'accroître le chemin optique sans pour autant augmenter le chemin à parcourir pour le VOC dans la matrice nanoporeuse. L'instrumentation peut être plus simple du fait du guidage de la lumière.

Le guide d'onde peut être de section transversale circulaire ou polygonale. Le guide d'onde peut présenter des faces d'entrée et de sortie de la lumière à l'opposé l'une de l'autre. Il est toutefois avantageux d'avoir des faces d'entrée et de sortie confondues, à une extrémité du guide d'onde, l'autre extrémité étant munie d'une surface réfléchissante. Cela peut simplifier la mise en place du guide d'onde dans l'espace où se situe le métabolite volatil VOC à détecter, les faces d'entrée et de sortie étant par exemple tournées vers un septum formant un réceptacle délimitant ledit espace.

Selon un autre mode de réalisation, le mode de détection du métabolite VOC est la détection en fluorescence. La matrice nanoporeuse associée est alors dénuée de fluorescence ou possède une faible fluorescence intrinsèque. L'adsorption et l'accumulation d'un métabolite volatil VOC fluorescent dans les pores de la matrice va se traduire par

une augmentation de la fluorescence de la matrice.

L'appareillage à considérer pour caractériser les propriétés optiques de la matrice, peut être tout appareillage conventionnel dédié à ce type de mesure.

A titre illustratif de ceux-ci peuvent notamment être cités les lampes et les spectroscopes.

Comme il ressort de ce qui suit, ces appareillages sont généralement disposés à l'extérieur de la matrice et du réservoir, mais ils sont optiquement couplés à la matrice. Une ou plusieurs fibres optiques permettent d'amener le signal d'excitation, mais aussi de collecter le signal de transduction. La matrice est dans le réacteur, par exemple couplée directement par collage à une ou plusieurs fibres optiques. En variante, le faisceau lumineux traverse la paroi du réacteur et la matrice nanoporeuse.

De préférence, la matrice est couplée directement par collage à au moins une fibre optique. Ce dispositif permet une meilleure sensibilité, en éliminant des sources de signal parasite, par exemple des réflexions à différentes interfaces.

On peut suivre l'évolution des propriétés optiques de la matrice sur toute une gamme de longueurs d'onde, auquel cas, on utilise de préférence un spectromètre UV-visible (absorbance) ou un spectrofluorimètre.

Pour suivre des changements de propriétés optiques de la matrice à une longueur d'onde donnée, on peut employer :

- une source, un filtre, un détecteur en absorbance, ou
- une source monochromatique, un système de collection de la fluorescence, et un détecteur.

Dispositif

On va maintenant décrire en référence au dessin annexé, des exemples de systèmes pour la mise en oeuvre du procédé selon l'invention.

Sur le dessin :

- la Figure 1 représente, de façon schématique et partielle, un exemple de système pour la mise en oeuvre du procédé selon l'invention,
- la Figure 2 représente, de façon schématique, une chaîne de détection,
- les Figures 3 et 4 représentent deux exemples d'agencement permettant de faciliter l'extraction des métabolites gazeux d'intérêt dans la phase liquide ou semi-solide du milieu de culture,
- la Figure 5 représente un exemple d'agencement d'enceinte de culture et de dispositif de mesure,
- la Figure 6 illustre la possibilité d'utiliser au sein d'un même système plusieurs enceintes contenant chacune un milieu de culture,
- la Figure 7 représente des substrats enzymatiques,
- les Figures 8 et 9 illustrent des résultats expérimentaux,
- la Figure 10 représente un guide d'onde utile pour la détection,
- la Figure 11 illustre la propagation d'un rayon lumineux dans le guide de la Figure 10,
- les Figures 12A et 12B représentent d'autres exemples de dispositifs de détection,
- les Figures 13A et 13B illustrent l'utilisation du dispositif de détection de la Figure 12A, respectivement en milieu gazeux et en milieu liquide.

Le système 1 selon l'invention, représenté à la Figure 1, comporte une enceinte 2, transparente et préférentiellement sans fluorescence intrinsèque afin de ne pas perturber la lecture optique, contenant le milieu de culture M et, en communication gazeuse avec la phase liquide ou semi-solide du milieu de culture M, un dispositif de détection 3 qui sera décrit plus en détails par la suite.

Le dispositif de détection 3 comprend une matrice nanoporeuse telle que détaillée précédemment et fait, par exemple, appel, comme illustré, à une détection optique qui fait intervenir un faisceau lumineux incident I dont la variation de l'intensité renseigne sur la présence dans le milieu de culture d'un métabolite VOC.

La détection peut faire appel à une chaîne de détection 5, représentée schématiquement à la Figure 2, ou un signal s est comparé à un signal de référence $s_{rf}$ puis une information utile est délivrée en fonction de cette comparaison.

La détection s'effectue en utilisant une matrice nanoporeuse, comme détaillée précédemment, avec laquelle le métabolite VOC peut entrer en contact et dont au moins une propriété optique change en fonction de la quantité de métabolite VOC à laquelle la matrice a été exposée. Le changement de cette propriété optique entraîne une modification du signal lumineux $I_s$ sortant, après propagation au sein de la matrice. Ce signal lumineux sortant peut être détecté par tout capteur adapté, par exemple une cellule photoélectrique, ce qui fournit le signal s.

Le signal de référence $s_{rf}$ peut être le signal obtenu avec, pour un même signal lumineux incident $I_i$, un signal lumineux sortant $I_s$ après traversée de la matrice nanoporeuse avant exposition aux métabolites VOC.

Le traitement des signaux délivrés par les capteurs peut s'effectuer à l'aide de tout dispositif 10 de traitement

adapté, par exemple microordinateur équipé des interfaces de conversion analogique numérique adaptées.

L'information délivrée par le circuit de traitement 10 peut être sous toutes formes, par exemple sous forme de message alphanumérique.

Sur la Figure 1, on a schématiquement représenté le dispositif de détection 3 au-dessus du milieu de culture M, étant enfermé dans la même enceinte 2 qui permet d'éviter les échanges gazeux avec l'extérieur.

Toute communication gazeuse entre l'enceinte dans laquelle est situé le milieu de culture et celle dans laquelle s'effectue la détection peut être envisagée, par exemple à l'aide de conduit(s) permettant une circulation de gaz entre la phase liquide ou semi-solide du milieu de culture et l'enceinte dans laquelle la détection s'effectue. La circulation gazeuse peut s'effectuer de façon naturelle ou, en variante être forcée, par exemple grâce à l'emploi d'une pompe ou d'un piston de tout mécanisme de brassage de gaz.

Une solution pour favoriser l'extraction des métabolites VOC de la phase liquide du milieu de culture peut consister, comme illustré à la Figure 3, à faire buller le gaz en l'amenant dans le milieu de culture à l'aide d'un conduit 12, ce conduit pouvant déboucher sur un diffuseur 13 immergé, de préférence à proximité du fond de l'enceinte contenant le milieu de culture.

L'enceinte renferme, de préférence, comme illustré sur la Figure 3, un mélangeur 15, par exemple à barreaux, qui permet de brasser la phase liquide d'un milieu de culture.

L'extraction du milieu gazeux au-dessus de l'enceinte peut s'effectuer par l'intermédiaire d'un conduit 16 qui débouche au-dessus de la surface 17 du milieu de culture.

Une autre façon d'accroître les échanges gazeux entre le milieu de culture et l'environnement gazeux au-dessus du milieu de culture peut consister à, comme illustré à la Figure 4, pomper le milieu de culture puis refouler ce milieu de culture au-dessus de la surface 17 de celui-ci, en le faisant s'écouler sur tout élément 19 adapté à accroître la surface d'échange, par exemple disposé sur une grille 20 au-dessus de la surface 17 du milieu de culture M.

On a représenté sur la Figure 5, une réalisation dans laquelle un réceptacle qui contient l'enceinte contenant le milieu de culture accueille à la fois ce dernier et le dispositif de détection.

Le milieu de culture M est par exemple contenu comme illustré dans un tube 25, lequel est disposé au-dessus d'une matrice nanoporeuse 30 destinée à réagir r optiquement avec le ou les VOC d'intérêt(s).

La matrice se présente par exemple sous la forme d'un bloc qui est disposé de façon à être traversé par un faisceau de lumière incident $I_i$.

La communication gazeuse entre la matrice 30 et le milieu de culture M peut s'effectuer par exemple par un espace 33 ménagé entre l'enceinte et la paroi du réceptacle 2.

On peut avantageusement utiliser au sein d'un système selon l'invention plusieurs enceintes 2 contenant chacune un milieu de culture, et communiquant chacune avec un dispositif de détection associé, par exemple comme illustré sur la Figure 6, de façon à permettre une identification phénotypique par mise en présence d'une souche microbienne et de différents substrats enzymatiques émettant tous le même métabolite volatil, mais ciblant différentes enzymes.

On peut également utiliser un dispositif pour la numération par MPN (Most Probable Number) et un dispositif pour l'AST (test de susceptibilités aux antibiotiques).

La méthode du nombre le plus probable (MPN, pour Most Probable Number) est employée pour compter des entités discrètes faciles à détecter, mais difficiles à énumérer (par exemple des bactéries dans un échantillon de sol, de sang, ou dans une matrice alimentaire).

La méthode de numération de microorganismes par MPN comporte les étapes consistant à:

1) Prendre l'échantillon original et le subdiviser en sous-volumes par ordres de grandeur (puissances de 10 ou de 2 le plus fréquement). La répartition des microorganismes dans les sous-volumes doit être aléatoire; cela implique donc une répartition homogène des cellules dans l'échantillon de départ juste avant la subdivision.
2) Interroger chacun des sous-volumes pour y déterminer la présence ou l'absence de microorganisme. Cette étape consiste dans notre cas à déterminer s'il y a une quantité non négligeable, ou pas, de métabolite VOC cible.
3) Connaissant le nombre de sous-volumes et les résultats (+ ou -) dans chacun d'entre eux, la loi de Poisson permet de remonter à la valeur la plus probable de la concentration en microorganismes dans l'échantillon original, ainsi qu'à un intervalle de confiance sur la mesure de concentration.

Pour le test AST (Antibiotic Susceptibility Testing), les étapes consistent à:

1) Subdiviser l'échantillon original en sous-échantillons de volumes identiques.
2) Incuber ensuite chacun d'entre eux en présence d'antibiotique. Différents antibiotiques sont testés, en général un par famille d'antibiotiques (macrolides, becta-lactames, fluoroquinolones, etc). En outre, pour un antibiotique donné, différentes concentrations sont testées.
3) De nouveau, interroger chaque sous-échantillon pour déterminer s'il y a eu, ou pas, croissance microbienne. Le

résultat consiste à donner les antibiotiques auxquels l'espèce testée est sensible, ainsi que la concentration minimale d'antibiotique nécessaire à l'inhibition de la croissance (MIC, Minimum Inhibitory Concentration; 1 valeur de MIC par antibiotique).

Chaque dispositif de détection 3, qui est en communication gazeuse avec le milieu de culture correspondant, permet de détecter s'il y a dégradation ou non du substrat enzymatique.

La matrice nanoporeuse 30 peut être réalisée sous forme de guide d'onde, comme illustré aux Figures 10 et 11.

Selon ce mode de réalisation, la matrice nanoporeuse a de préférence une forme cylindrique, et son indice de réfraction est supérieur à celui du milieu extérieur, afin qu'elle se comporte en guide d'onde. Cette matrice est illuminée par un faisceau lumineux d'intensité $I_0$, ou faisceau incident. On mesure alors l'intensité lumineuse I transmise par le guide d'onde.

L'interaction du VOC produit une absorption de la lumière du guide d'onde, qu'il est possible de mesurer en comparant l'intensité incidente et l'intensité transmise. Une comparaison est par exemple la détermination de la grandeur $\log(I_0/I)$ = e L c où e est le coefficient d'extinction molaire ($Mol^{-1}.1.cm^{-1}$), L le chemin optique (cm), et c la concentration de l'espèce absorbante crée par l'interaction du VOC dans la matrice poreuse ($mol.1^{-1}$).

$I_0$ peut être déterminée avant l'absorption, ou par une voie de mesure indépendante, et donc, d'une façon générale, par une mesure de référence.

Dans ces conditions, la mesure de I (intensité du faisceau transmis) permet d'estimer c, les grandeurs e et L étant connues.

On peut disposer, comme illustré aux Figures 12A et 12B, une surface formant un miroir 50 à une extrémité du guide, située à l'opposée de la face d'entrée par laquelle entre le signal d'intensité $I_0$. Le miroir est soit plan comme illustré à la Figure 12A, soit arrondi comme illustré à la Figure 12B. Cela a pour effet de doubler le chemin optique par rapport à la configuration de la Figure 10. On augmente alors la sensibilité de la mesure car pour une concentration c donnée, l'atténuation mesurée, quantifiée par log $I_0/I$, est plus importante.

Dans les configurations des Figures 12A et 12B, tout comme celle de la Figure 10, c'est la variation dans le temps du signal lumineux de retour qui permet la détection : il n'y a pas de faisceau de référence.

La référence $I_i$ désigne le signal incident et $I_r$ le signal retour. Dans l'exemple de la Figure 12A, le miroir 50 est par exemple en silicium. Dans la Figure 12B, l'extrémité arrondie 51 qui réfléchit la lumière est par exemple préparée par moulage de la matrice 30 dans un tube à fond rond. La lumière peut être réfléchie compte-tenu des lois de la réfraction, grâce à la différence d'indice.

Ainsi, selon un mode de réalisation préféré, la détection optique s'effectue après un aller-retour d'un faisceau incident dans un guide d'onde formé par la matrice ou après une traversée d'un guide d'onde formé par la matrice, le guide d'onde étant par exemple muni en cas d'aller-retour du faisceau incident d'un miroir (50), de préférence en silicium, ou d'une extrémité arrondie (51), de préférence hémisphérique.

La matrice nanoporeuse 30 peut être disposée dans la phase gazeuse comme illustré à la Figure 13A ou dans le milieu nutritif comme illustré à la Figure 13B.

Dans l'exemple des Figures 13A et 13B, le signal d'excitation est amené par au moins une fibre optique et le signal après parcours dans la matrice nanoporeuse 30 est transmis par au moins une fibre. Les fibres traversent un septum 31, qui bouche un flacon 2 définissant l'enceinte contenant le milieu de culture M.

Le guide d'onde est par exemple orienté verticalement. La ou les fibres optiques servant à véhiculer les signaux incident et de retour sont par exemple collée(s) à une extrémité du guide d'onde, par exemple l'extrémité supérieure comme illustré.

De préférence, la matrice nanoporeuse n'est plongée dans la phase liquide que si:

a) les composés colorés ou diffusants de la phase liquide ne viennent pas perturber outre mesure la mesure optique dans la matrice;
b) d'autres solutés (tampons de pH par exemple), susceptibles de rentrer dans les nanopores, ne perturbent pas outre mesure la mesure optique ;
c) la sonde éventuellement immobilisée dans la matrice ne quitte pas les nanopores pour aller diffuser dans la phase liquide.

L'invention a encore pour objet, selon l'un de ses aspects, un système dédié à la détection d'un microorganisme dans un milieu biologique comportant une enceinte pour recevoir un milieu de culture, un dispositif de détection d'un métabolite VOC libéré par métabolisation d'un substrat enzymatique par le microorganisme, ce système de détection étant en communication gazeuse avec ledit milieu de culture ou directement en contact.

L'enceinte contenant le milieu de culture peut être de volume suffisamment grand pour accueillir également le système de détection au moins partiellement.

Dans un exemple de mise en oeuvre de l'invention, le milieu de culture est contenu dans un récipient qui se

situe au-dessus d'une matrice nanoporeuse exposée aux métabolites VOC, et dont les propriétés optiques sont sensibles à la présence du métabolite VOC.

Cette matrice nanoporeuse est contenue dans une enceinte adaptée à être traversée par un faisceau lumineux incident permettant d'en mesurer l'absorbance au cours du temps, en fonction de la quantité de métabolite VOC ayant réagi avec la matrice.

La matrice nanoporeuse peut se présenter sous forme de guide d'onde. La matrice nanoporeuse peut être parcourue selon un aller-retour par un faisceau de lumière incident, ce faisceau pouvant se réfléchir sur une surface réfléchissante plane ou concave. La surface réfléchissante peut être définie par un miroir en silicium.

Le système peut comporter une circulation de gaz avec prélèvement au-dessus du milieu de culture et réinjection dans le milieu de culture, de préférence par l'intermédiaire d'un diffuseur.

Dans un autre exemple de mise en oeuvre de l'invention, le système comporte une circulation du milieu de culture avec prélèvement du milieu de culture et rejet au-dessus d'au moins un élément adapté à accroître la surface d'échanges gazeux entre le milieu de culture et l'environnement gazeux, de préférence un lit d'éléments, tel que des billes sur lequel le milieu de culture est déversé.

Un dispositif conforme à l'invention peut comprendre :

- un flacon en verre (matériau non absorbant, non fluorescent) ;
- un septum pour l'injection du prélèvement dans le milieu nutritif ;
- un milieu nutritif contenant:
  - une source de C
  - une source de N
  - des sels minéraux
  - des oligo-éléments
  - un (des) substrat(s) enzymatique(s)
  - un tampon de pH
  - éventuellement des adsorbants pour la capture des antibiotiques résiduels
  - éventuellement des inhibiteurs de croissance pour rendre le milieu spécifique
- un ou des capteurs nanoporeux, placé(s) dans la phase gaz, pour la capture et la détection optique du (des) métabolite(s) volatil(s) VOC(s) émis par les microorganismes.

Ce capteur comporte, voire est constitué, par une matrice nanoporeuse, préférentiellement à base d'oxyde de silicium, avec une distribution de tailles adaptée à la capture spécifique du métabolite volatil VOC. On peut aussi envisager tout matériau poreux créé par polycondensation d'alcoolates de formule $M(X)_m(OR_a)_n(R_b)_p$ dans laquelle :

- M correspond à un métal choisi parmi le silicium, l'aluminium, le tungstène, le titane, le zirconium, le niobium, le vanadium, le tantale, le yttrium et le cérium,
- $R_a$ correspond à un radical alkyle en $C_1$ à $C_6$ ou à un aryle en $C_5$,
- $R_b$ correspond à un radical alkyle en $C_1$ à $C_6$, à un aryle en $C_5$ à $C_{10}$ ou à un aminoalkyle en $C_3$ à $C_6$,
- n, m et p sont des entiers, tels que leur somme est égale à la valence de M et n est supérieur ou égal à 2, m et p pouvant être égaux à 0, et
- X est un halogène, de préférence le chlore.

La taille des pores est inférieure à 100 nm, de préférence varie de 3 à 100 Å. La surface spécifique peut varier de 300 à 1000 $m^2/g$, préférentiellement de 300 à 900 $m^2/g$, et en particulier, de 400 à 900 $m^2/g$ ;

- un dispositif d'agitation mécanique, avec un système de barbotage ou de nébulisation ou de billes de verres: tout procédé permettant d'accélérer le transfert de masse du métabolite volatil VOC de la phase liquide vers la phase gaz ;
- une membrane imperméable aux liquides mais pas aux gaz, pour protéger le capteur des projections de liquide (dues à l'agitation) ;
- un spectrophotomètre d'absorbance UV-visible ou un spectrofluorimètre, extérieur au flacon.

Avantageusement, lès étapes 1) à 3) d'un procédé de l'invention peuvent être réalisées au sein d'un flacon, par exemple d'hémoculture.

On peut également envisager un dispositif où différentes enzymes sont testées sur une même souche afin de procéder à une identification phénotypique d'un microorganisme. Différentes cultures contenant différents substrats enzymatiques générant le même composé volatil, sont inoculées avec la même souche. Un capteur est placé dans

l'atmosphère de chaque culture de manière à détecter s'il y a une activité enzymatique ou non. Un tel dispositif est particulièrement avantageux pour le multi-dosage de microorganisme.

La présence de plusieurs substrats enzymatiques ciblant les principales voies enzymatiques présentes chez les mycobactéries peut permettre avantageusement la détection en phase gaz de ces microorganismes. Deux solutions technologiques se présentent :

- soit les substrats conduisent tous au même métabolite volatil VOC: on assure alors une simple détection ;
- soit les substrats forment différents métabolites volatils VOC avec des propriétés optiques distinctes : on peut alors procéder, si le nombre de substrats est suffisant, à une identification de la mycobactérie détectée.

Dans le cas par exemple de l'hémoculture, on pourra employer un mélange de plusieurs substrats enzymatiques ciblant les voies enzymatiques les plus courantes, de manière à pouvoir détecter n'importe lequel des pathogènes rencontrés en hémoculture.

Toutes les espèces de bactéries pathogènes susceptibles d'infecter le système sanguin doivent présenter au moins une des voies enzymatiques ciblées.

Le schéma en Figure 7 décrit l'exemple de trois substrats enzymatiques phénoliques émettant tous le même métabolite volatil VOC, le m-cyano-p-nitrophénol. Le mélange de ces 3 substrats dans un milieu de culture permet de cibler 3 enzymes : la ß-galactosidase, l'estérase et la phosphatase alcaline. On peut ainsi détecter toute espèce de bactérie qui présente au moins l'une de ces 3 enzymes.

Au sens de l'invention, sauf indication contraire, « un » signifie « au moins un» et l'expression «compris entre ... et ... » inclut, dans l'intervalle ainsi défini, les bornes.

Les exemples indiqués ci-après sont présentés à titre d'illustration de l'invention, sans caractère limitatif.

**Exemples**

Exemple 1 - Synthèse d'un monolithe nanoporeux doté d'une affinité pour le p-nitrophénol, pNP

Cet exemple illustre la synthèse d'une matrice basique Sol "mixte" $Si(OMe)_4$-$NH_2$TEOS. Le protocole de synthèse retenu est le procédé par voie sol-gel d'une matrice nanoporeuse pour la capture d'espèces aromatiques monocycliques, illustré dans la demande WO 2010/004225.

*Réactifs de départ :*

Précurseurs :

TMOS (tetraméthoxysilane) (= $Si(OMe)_4$)
APTES, $Si(C_3H_6H_6NH_2)(OC_2H_5)_3$ ((3-aminopropyl)triethoxysilane)

Solvant:

MeOH (methanol)
Hydrolyse avec eau ultrapure

*Rapports molaires :*

Alkoxydes/méthanol/eau : 1/5/4
TMOS/APTES : 0,97/0,03

Proportions pour 5mL environ de sol $NH_2$TEOS à 3 %
TMOS 1,786 mL
MeOH 2,43 mL
APTES 0,084 mL
Eau 0,864 mL

*Protocole :*

TMOS et MeOH sont mélangés sous agitation magnétique pendant 2 min dans un bécher pyrex placé dans un bain à -25 °C (éthanol et azote liquide).

L'APTES est ajouté à la micropipette et l'ensemble est agité pendant 2 min.

L'eau ultrapure y est ajoutée et l'ensemble est agité pendant 30 s.

Les matrices sont mises en forme rapidement car le sol gèle vite (1mL dans une cuve, les cuves ayant été prétraitées à 50°C pendant 24 h en dégazant l'étuve au moins à trois reprises pendant cette durée).

Les cuves sont obturées avec leur bouchon et laissées 24 heures. Les bouchons sont ensuite ôtés et remplacés par un film microporeux pour laisser évaporer les solvants.

Exemple 2

Une activité β-glucuronidase est ciblée sur deux modèles biologiques, à savoir *Escherichia coli* ATCC 11775 (β-glucuronidase positive) et *Hafnia alvei* ATCC 13337 (β-glucuronidase négative).

Le métabolite volatil VOC choisi est le p-nitrophénol ($pK_a$ = 7,15).

La matrice retenue est celle décrite en exemple 1. Elle permet l'accumulation de pNP sous forme $pNP^-$.

Le tableau ci-après détaille la composition du milieu de culture "MES-pNPG" générant du p-nitrophénol par activité β-glucuronidase.

Ce milieu de culture est appelé "MES-pNPG" car le MES en est le tampon de pH, et le pNPG (4-nitrophényl-β-D-glucuronide) en est le substrat enzymatique.

Le pH est vérifié après étalonnage (3 points : 4, 7 et 10), il est égal à 6,09.

| **Composant** | | **Concentration** |
|---|---|---|
| Milieu nutritif (avec sels minéraux et éléments trace) | bio-soyase | 2 g/L |
| | extraits de levure | 2 g/L |
| | NaCl | 5 g/L (86 mM) |
| | $MgSO_4$ | 250 mg/L (2,08 mM) |
| Tampon de pH | MES sel de sodium | 16,292 $g.L^{-1}$ (75 mM) |
| | MES acide monohydrate | 15,994 $g.L^{-1}$ (75 mM) |
| Inducteurs de l'activité enzymatique β-D-glucuronidase | glucuronate de sodium monohydrate | 108 $mg.L^{-1}$ (461 μM) |
| | methyl β-D-glucuronide | 55,2 $mg.L^{-1}$ (240 μM) |
| Substrat enzymatique de l'enzyme β-D-glucuronidase selon l'invention | pNPG (4-nitrophényl-β-D-glucuronide) | 35,6 $mg.L^{-1}$ (113 μM) |

Pour imposer sur une forme acide majoritaire du paranitrophénol, le milieu de culture est tamponné à $pK_a$-1, soit 6,15, avec du MES (l'acide 4-morpholinoéthanesulfonique).

2.1 - Contrôle témoin de l'activité enzymatique

Préliminairement, il est procédé à un contrôle de l'activité enzymatique et de la croissance bactérienne dans la phase liquide constituée par le milieu de culture.

L'objectif est de vérifier au préalable, dans la phase liquide, la formation effective du métabolite volatil VOC absorbant pNP et de le quantifier (μM) par spectroscopie d'absorbance à 400 nm. On suit également la population bactérienne (cfu/mL) en fonction du temps, de manière à déterminer le temps de génération du modèle bactérien dans le milieu de culture préparé (MES-pNPG).

La préculture est une colonie d'*Escherichia coli* 5 (*Escherichia coli* ATCC 11775) sur une boîte TSA qui est suspendue dans 4 mL de milieu de culture LB (Lysogenic Broth), sous agitation.

La préculture est conservée à 30 °C en statique pendant 14h.

La culture est mise dans un milieu de culture LB (Lysogenic Broth), puis en milieu MES-pNPG tel que décrit précédemment.

Le tube de préculture le plus concentré possède un degré d'absorbance (DO) de : DO(1/2x) = 0,5685, soit un degré d'absorbance effectif de 1,137.

L'inoculation se fait sur une base de 1/50ème pour un degré d'absorbance de 0,7 donc: (1,1137/0,7)x50=81,2. L'inoculation se fait donc au 1/81ème, soit 49 μL pour un volume total de 4 mL.

On inocule donc 2 tubes de 4 mL de LB à 37 °C, à 250 rpm pendant 2h et 25 minutes. On mesure

DO550(1/2x)=0,35, soit 0,7 de degré d'absorbance effectif.

On inocule (t=0) le tampon MES-pNPG au 1/700ème : 20 μL de culture de EC5 en LB à 0,7 de degré d'absorbance pour 14 mL de MES-pNPG.

L'incubation se fait à 37 °C, à 250 rpm.

Pour chaque prélèvement :

- 0,5 mL sont prélevés pour une dilution en cascades dans le sérum physiologique et un étalement sur boîte LB agar ;
- 1,5 mL servent à mesurer la concentration bactérienne avec le DensiCheck® ;
- Après mesure au DensiCheck®, les 1,5 mL utilisés sont filtrés sur Acrodisc® 0,2 μm et versés dans une cuve plastique pour la spectro UV-vis (balayage : 200 nm/min, de 600 à 300 nm, avec cuve d'eau milli Q pour référence) : le premier spectre est réalisé ;
- Ajout de 80 μL de NaOH 5M pour neutraliser le tampon MES et faire passer tous les pNP en anions phénolates pNP- : on réalise alors un deuxième spectre (en effet, le pic du pNP- étant décalé vers les grandes longueurs d'onde il est plus facile de quantifier l'absorbance ; en outre l'extinction molaire du pNP- est plus grande).

Le degré d'absorbance initial de 0,7, qui est ensuite diluée à t=0 au 1/760ème, correspond à $1,54.10^8$ cfu/mL.

Le tampon MES-pNPG comporte donc $2,2.10^5$ cfu/mL à t=0.

La Figure 8 témoigne effectivement de la croissance et du suivi de l'activité enzymatique β-glucuronidase pour une culture d'*Ercherichia coli* inoculée à $2.10^5$ cfu/mL (à 37 °C) dans le tampon MES-pNPG. Le temps de génération lors de la phase exponentielle est de 24 minutes.

2.2 - Mise en oeuvre du procédé selon l'invention pour caractériser la présence du microorganisme *Escherichia coli* dans le milieu de culture

L'objectif est ici de détecter, dans la phase gaz, le VOC issu d'une culture en milieu MES-pNPG à l'aide d'un monolithe nanoporeux.

Le dispositif retenu, permet de suivre dans le temps l'absorbance du monolithe, entre 300 et 600 nm (UVIKON 933, Double Beam UV/Vis Spectrophotometer, KONTRON Instruments) sans ouvrir le récipient clos dans lequel la culture et le monolithe sont mis en présence. Ce dispositif est illustré en Figure 5.

La préculture est une colonie d'*Escherichia coli* 5 (*Escherichia coli* ATCC 11775) sur une boîte TSA (boîte 100802_CT4 d'EB) qui est suspendue dans 4 mL de LB sans ampicilline (x2 tubes), sous agitation.

La préculture est conservée à 30 °C en statique pendant 13h et 30 minutes.

La culture est mise en LB, puis en tampon MES-pNPG.

Le tube de préculture le plus concentré possède un degré d'absorbance de DO(1/2x) = 0,52, soit une DO effective de 1,04.

L'inoculation se fait sur une base de 1/50ème pour un degré d'absorbance de 0,7 donc: (1,04/0,7)x50=74. On inocule donc au 1/74ème, soit 54 μL pour un volume total de 4 mL.

On inocule donc 2 tubes de 4 mL de LB sans ampicilline.

L'incubation est faite pendant 1h et 30 minutes à 37 °C, à 250 rpm.

On mesure un degré d'absorbance de DO550(1x)=0,30 (soit ~$6.10^7$ cfu/mL).

0,5 mL sont prélevés pour une dilution en cascades jusque $10^{-5}$ pour réaliser une numération sur boîte : $6,2.10^7$ cfu/mL.

On inocule le tampon MES-pNPG au 1/600ème: 8,5 μL de culture d'*Escherichia coli 5* en LB à 0,3 de DO pour 5 mL de MES-pNPG.

1,5 mL de cette culture vont dans le montage fermé destiné aux mesures spectroscopiques, le reste est incubé dans un tube classique de 15 mL.

La culture de la bactérie *Escherichia coli* 5 dans le milieu de culture enzymatique est réalisée dans un tube enfermé dans une enceinte étanche en présence d'un monolithe nanoporeux basique. Le suivi cinétique de l'absorbance est réalisé sur le monolithe, au travers de la paroi de l'enceinte (une cuve de spectrophotométrie fermée). On met donc sur le faisceau de référence une cuve vide, de manière à compenser l'absorbance éventuelle du plastique de la cuve.

L'incubation se fait à 37 °C, à 150 rpm (t=0).

Le monolithe employé est bien transparent et incolore avant le début de l'expérience. Le degré d'absorbance à 375 nm du monolithe à t=0 est de 0,25.

A t=9h et 15 minutes, on sort le dispositif de l'étuve pour le laisser dans le spectro (300 à 600 nm, data interval 1 nm, scan speed 200 nm/min ; 10 cycles, cycle time 80 min (1h20), 1 sample).

En parallèle, un comptage sur boîte est réalisé sur le tube qui contenait le même inoculum et qui a été incubé en parallèle, cela permet une estimation de la population.

Le graphique de la Figure 9 présente l'augmentation d'absorbance à 375 nm du monolithe nanoporeux exposé

aux vapeurs de pNP (culture d'*Escherichia coli* 5 à 37 °C en MES-pNPG, inoculée à t=0 avec $10^5$ cfu/mL).

## Revendications

1. Procédé pour révéler la présence ou l'absence d'au moins un microorganisme dans un milieu biologique, ledit procédé comprenant au moins les étapes consistant à :

   1) disposer d'une enceinte contenant :

      - une phase liquide ou semi-solide formée en tout ou partie dudit milieu biologique susceptible de contenir au moins une forme vivante dudit microorganisme, des éléments nutritifs nécessaires à la prolifération dudit microorganisme, et d'un substrat enzymatique spécifique audit microorganisme et métabolisable en au moins un métabolite volatil VOC, et
      - une phase gazeuse contiguë à ladite phase liquide ou semi-solide,

   2) exposer au moins ladite phase liquide ou semi-solide à des conditions propices à la métabolisation dudit substrat enzymatique par ledit microorganisme, en au moins une molécule dudit métabolite volatil VOC, et
   3) révéler, par transduction optique, la présence ou l'absence dudit métabolite VOC, indicateur de la présence dudit microorganisme,

   **caractérisé en ce que** :

   - ledit métabolite VOC, si formé en étape 2), interagit avec une matrice nanoporeuse, ladite matrice étant mise en oeuvre sous une forme séparée dudit substrat enzymatique, et
   - la détection par transduction optique d'un changement des propriétés optiques de ladite matrice, est indicatrice d'une interaction de ladite matrice avec ledit métabolite.

2. Procédé selon la revendication précédente, **caractérisé en ce que** ledit métabolite est doté de propriétés optiques intrinsèques.

3. Procédé selon la revendication 1 ou 2, **caractérisé en ce que** ledit métabolite VOC est distinct des métabolites naturels générés lors d'une croissance microbienne.

4. Procédé selon l'une quelconque des revendications précédentes, **caractérisé en ce que** ladite matrice nanoporeuse est disposée en phase gazeuse ou phase liquide dans ladite enceinte.

5. Procédé selon l'une quelconque des revendications précédentes **caractérisé en ce que** la détection par transduction optique est réalisée au niveau de ladite matrice nanoporeuse disposée en phase gazeuse.

6. Procédé selon l'une quelconque des revendications précédentes, **caractérisé en ce que** ledit substrat enzymatique est un substrat phénolique ou naphtolique.

7. Procédé selon l'une quelconque des revendications précédentes, **caractérisé en ce que** ledit métabolite VOC est choisi parmi les dérivés phénoliques, tels que les dérivés nitrophénoliques, cyanophénoliques, cyanonitrophénoliques, acétylphénoliques, propionylphénoliques, les dérivés thiophénoliques, les dérivés naphtoliques, les dérivés aniliques, tels que les dérivés nitroaniliniques, ou les dérivés naphtylaminiques et en particulier est choisi parmi le p-cyanophénol, le 4-nitrophénol (p-nitrophénol), le m-cyano-p-nitrophénol (3-cyano-4-nitrophénol), le 2,6-dichloro-4-nitrophénol, le 2,6-dichloro-4-acetylphénol, le thiophénol, le 2,6-dichloro-4-propionylphénol, le 2,6-difluoro-4-acétylphénol, le 2,6-dibromo-4-acétylphénol, le 1-naphtol, le 2-naphtol, l'$\alpha$-naphthylamine, la $\beta$-naphthylamine, la 4-nitroaniline (p-nitroaniline), l'ombelliférone, la naphthazarine, la 4-trifluorométhylombelliférone, la 4-méthylombelliférone, le o-cyanophénol, le m-cyanophénol, le 6-cyano-2-naphtol, le 6-hydroxyquinoline-N-oxyde, le 2-méthyl-6-hydroxyquinoline-N-oxyde, la 6-hydroxyquinoline, la 7-hydroxyquinoline, ou la 8-hydroxyquinoline.

8. Procédé selon l'une quelconque des revendications précédentes, **caractérisé en ce que** le métabolite VOC est un photoacide ou une photobase.

9. Procédé selon l'une quelconque des revendications précédentes, **caractérisé en ce que** ledit métabolite VOC est

détectable en absorbance et/ou en fluorescence.

**10.** Procédé selon l'une quelconque des revendications précédentes, **caractérisé en ce que** ledit métabolite VOC est détectable en absorbance, et **en ce que** la matrice nanoporeuse associée est transparente ou peu absorbante dans la zone de détection.

**11.** Procédé selon l'une quelconque des revendications précédentes, **caractérisé en ce que** ledit métabolite VOC est détectable en fluorescence, et **en ce que** la matrice nanoporeuse associée est dénuée de fluorescence intrinsèque ou possède une faible fluorescence intrinsèque.

**12.** Procédé selon l'une quelconque des revendications précédentes, **caractérisé en ce que** ladite matrice possède une distribution de taille de pores ajustée à la taille dudit métabolite VOC, et en particulier inférieure à 100 nm, et de préférence variant de 3 à 100 Å.

**13.** Procédé selon l'une quelconque des revendications précédentes **caractérisé en ce que** ladite matrice possède une surface spécifique variant de 300 à 1000 $m^2.g^{-1}$, voire de 300 à 900 $m2.g^{-1}$, et en particulier de 400 à 900 $m^2.g^{-1}$.

**14.** Procédé selon l'une quelconque des revendications précédentes, **caractérisé en ce que** ladite matrice est constituée d'un matériau organique, inorganique ou hybride organique-inorganique.

**15.** Procédé selon l'une quelconque des revendications précédentes, **caractérisé en ce que** ladite matrice dérive de la polycondensation d'alcoolates de formule

$$M(X)_m(OR_a)_n(R_b)_p,$$

dans laquelle :

- M correspond à un métal choisi parmi le silicium, l'aluminium, le tungstène, le titane, le zirconium, le niobium, le vanadium, le tantale, le yttrium et le cérium,
- $R_a$ correspond à un radical alkyle en Cl à $C_6$ ou à un aryle en $C_5$,
- $R_b$ correspond à un radical alkyle en $C_1$ à $C_6$, à un aryle en $C_5$ à $C_{10}$ ou à un aminoalkyle en $C_3$ à $C_6$,
- n, m et p sont des entiers, tels que leur somme est égale à la valence de M et n est supérieur ou égal à 2, m et p pouvant être égaux à 0, et
- X est un halogène, de préférence le chlore.

**16.** Procédé selon l'une quelconque des revendications précédentes, **caractérisé en ce que** ladite matrice comprend au moins une molécule sonde apte à amplifier le signal de transduction optique via son interaction avec ledit métabolite VOC piégé dans ladite matrice, ladite molécule sonde étant en particulier choisie parmi le chlorure de 4-benzoylamino-2,5-diéthoxybenzènediazonium (Fast Blue BB), le diméthylaminocinnamaldéhyde (DMACA) et le 5,5'-dithio-bis-(2-nitrobenzoic acid), dit réactif de Ellman.

**17.** Procédé selon l'une quelconque des revendications précédentes, **caractérisé en ce que** ladite matrice est disposée en phase gazeuse et **en ce que** l'étape 2) met en oeuvre des moyens optimisant la circulation dudit métabolite volatil VOC vers la phase gazeuse.

**18.** Procédé selon la revendication précédente, **caractérisé en ce que** les moyens optimisants sont figurés par une agitation mécanique ou une nébulisation en surface de la phase liquide ou semi-solide, et en particulier sont figurés par ruissellement de la phase liquide sur une phase solide inerte divisée, telle qu'une accumulation de billes de verre, afin d'accroître la surface de l'interface liquide-gaz.

**19.** Utilisation du procédé selon l'une quelconque des revendications précédentes pour détecter la présence de microorganisme(s) dans un milieu biologique et de préférence dans un milieu physiologique.

**20.** Utilisation du procédé selon l'une quelconque des revendications 1 à 18, pour effectuer un test antibiogramme (AST).

**Patentansprüche**

1. Verfahren zur Feststellung der Anwesenheit oder Abwesenheit wenigstens eines Mikroorganismus in einem biologischen Milieu, welches Verfahren wenigstens die folgenden Schritte umfasst:

    1) Bereitstellen eines Gehäuses, das enthält:

        - eine flüssige oder halbfeste Phase, die ganz oder zum Teil durch das genannte biologische Milieu, das im Verdacht steht, wenigstens eine lebende Form des betreffenden Mikroorganismus zu enthalten, Nährstoffe, die zur Vermehrung dieses Mikroorganismus notwendig sind, und ein enzymatisches Substrat gebildet wird, das für den Mikroorganismus spezifisch ist und in wenigstens einen flüchtigen Metaboliten VOC metabolisierbar ist, und
        - eine an die flüssige oder halbfeste Phase angrenzende gasförmige Phase,

    2) Einwirkenlassen von Bedingungen, die für den Metabolismus des genannten enzymatischen Substrats durch den genannten Mikroorganismus in wenigstens ein Molekühl des genannten volatilen Metaboliten VOC günstig ist, auf die genannte flüssige oder halbfeste Phase, und
    3) Feststellen, durch optische Transduktion, der Anwesenheit oder Abwesenheit des genannten Metaboliten VOC als Indikator für die Anwesenheit des Mikroorganismus,

    **dadurch gekennzeichnet, dass**:

    - der Metabolit VOC, wenn er in Schritt (2) gebildet wird, mit einer nanoporösen Matrix wechselwirkt, die in einer von dem genannten enzymatischen Substrat getrennten Form bereitgestellt wird, und
    - die Detektion einer Änderung der optischen Eigenschaften der genannten Matrix durch optische Transduktion ein Indikator einer Wechselwirkung dieser Matrix mit dem Metaboliten ist.

2. Verfahren nach dem vorstehenden Anspruch, **dadurch gekennzeichnet**, das der Metabolit mit intrinsischen optischen Eigenschaften ausgestattet ist.

3. Verfahren nach Anspruch 1 oder 2, **dadurch gekennzeichnet**, das der Metabolit VOC von natürlichen Metaboliten verschieden ist, die bei einem Mikrobenwachstum erzeugt werden.

4. Verfahren nach einem der vorstehenden Ansprüche, **dadurch gekennzeichnet, dass** die nanoporöse Matrix innerhalb des Gehäuses in der gasförmigen Phase oder flüssigen Phase angeordnet ist.

5. Verfahren nach einem der vorstehenden Ansprüche, **dadurch gekennzeichnet, dass** die Detektion durch optische Transduktion an der nanoporösen Matrix vorgenommen wird, die in der gasförmigen Phase angeordnet ist.

6. Verfahren nach einem der vorstehenden Ansprüche, **dadurch gekennzeichnet, dass** das enzymatische Substrat ein phenolisches oder naphtolisches Substrat ist.

7. Verfahren nach einem der vorstehenden Ansprüche, **dadurch gekennzeichnet, dass** der Metabolit VOC ausgewählt ist aus phenolischen Derivaten wie etwa nitrophenolischen, cyanophenolischen, cyanonitrophenolischen, acetylphenolischen, propionylphenolischen Derivaten, thiophenolischen Derivaten, naphtolischen Derivaten, Anilinderivaten wie etwa Nitroanilinderivaten oder Naphtylaminderivaten und insbesondere ausgewählt ist unter p-Cyanophenol, 4-Nitrophenol(p-Nitrophenol), m-Cyano-p-Nitrophenol(3-Cyano-4-Nitrophenol), 2,6-Diclor-4-Nitrophenol, 2,6-Dichlor-4-Acetylphenol, Thiophenol, 2,6-Dichlor-4-Propinonylphenol, 2,6-Difluor-4-Acetylphenol, 2,6-Dibrom-4-Acetylphenol, 1-Naphtol, 2-Naphtol, $\alpha$-Naphtylamin, $\beta$-Naphtylamin, 4-Nitroanilin(p-Nitroanilin), Umbelliferon, Naphtazarin, 4-Trifluormethylumbelliferon, 4-Methylumbelliferon, o-Cyanophenol, m-Cyanophenol, 6-Cyano-2-Naphtol, 6-Hydroxyquinolin-N-Oxid, 2-Methyl-6-Hydroxyquinolin-N-Oxid, 6-Hydroxyquinolin, 7-Hydroxyquinolin oder 8-Hydroxyquinolin.

8. Verfahren nach einem der vorstehenden Ansprüche, **dadurch gekennzeichnet, dass** der Metabolit VOC eine Photosäure oder eine Photobase ist.

9. Verfahren nach einem der vorstehenden Ansprüche, **dadurch gekennzeichnet, dass** der Metabolit VOC in Absorption und/oder Fluoreszenz detektierbar ist.

**10.** Verfahren nach einem der vorstehenden Ansprüche, **dadurch gekennzeichnet, dass** der Metabolit VOC in Absorption detektierbar ist und dass die zugehörige nanoporöse Matrix in der Detektionszone transparent oder wenig absorbierend ist.

**11.** Verfahren nach einem der vorstehenden Ansprüche, **dadurch gekennzeichnet, dass** der Metabolit VOC in Fluoreszenz detektierbar ist und dass die zugehörige nanoporöse Matrix keine intrinsische Fluoreszenz aufweist oder eine geringe intrinsische Fluoreszenz besitzt.

**12.** Verfahren nach einem der vorstehenden Ansprüche, **dadurch gekennzeichnet, dass** die Matrix eine Größenverteilung der Poren aufweist, die auf die Größe des Metaboliten VOC eingestellt ist, insbesondere kleiner als 100 nm, vorzugsweise variierend zwischen 3 bis 100 Angström.

**13.** Verfahren nach einem der vorstehenden Ansprüche, **dadurch gekennzeichnet, dass** die Matrix eine spezifische Oberfläche aufweist, die zwischen 300 bis 1000 $m^2g^{-1}$ variiert, bevorzugt von 300 bis 900 $m^2g^{-1}$ und insbesondere von 400 bis 900 $m^2g^{-1}$.

**14.** Verfahren nach einem der vorstehenden Ansprüche, **dadurch gekennzeichnet, dass** die Matrix durch ein organisches oder anorganisches Material oder ein organischanorganisches Hybridmaterial gebildet wird.

**15.** Verfahren nach einem der vorstehenden Ansprüche, **dadurch gekennzeichnet, dass** die Matrix aus der Polykondensation von Alcoolaten gebildet ist, mit der Formel

$$M(X)_m(OR_a)_n(R_b)_p,$$

wobei

- M ein Metall bedeutet, das ausgewählt ist unter Silizium, Aluminium, Wolfram, Titan, Zirkonium, Niob, Vanadium, Tantal, Yttrium und Cer,
- $R_a$ ein $C_1$- bis $C_6$-Alkylradikal oder ein $C_5$-Aryl bedeutet,
- $R_b$ ein $C_1$- bis $C_6$-Alkylradikal, ein $C_5$ bis $C_{10}$-Aryl oder $C_3$ bis $C_6$ Aminoalkyl bedeutet,
- n, m und p ganze Zahlen sind, derart, dass ihre Summe gleich der Valenz von M ist und n größer oder gleich 2 ist, wobei m und p gleich 0 sein können, und
- X ein Halogen, vorzugsweise Chlor ist.

**16.** Verfahren nach einem der vorstehenden Ansprüche, **dadurch gekennzeichnet, dass** die Matrix wenigstens ein Sondenmolekül enthält, das dazu geeignet ist, das optische Transduktionssignal durch seine Wechselwirkung mit dem in der Matrix verborgenen Metaboliten VOC zu verstärken, wobei das Sondenmolekül insbesondere ausgewählt ist unter Chloriden von 4-Benzoylamino-2,5-Diethoxybenzendiazon (Fast Blue BB), Dimethylaminocinnamaldehyd (DMACA) und 5,5'-Dithio-bis-(2-Nitrobenzolsäure), genannt Ellman-Reagens.

**17.** Verfahren nach einem der vorstehenden Ansprüche, **dadurch gekennzeichnet, dass** die Matrix in der gasförmigen Phase angeordnet ist und dass der Schritt 2) Mittel benutzt, die den Strom des volatilen Motaboliten VOC in die gasförmig Phase optimieren.

**18.** Verfahren nach dem vorstehenden Anspruch, **dadurch gekennzeichnet, dass** die optimierenden Mittel gebildet werden durch mechanisches Rühren oder eine Vernebelung an der Oberfläche der flüssigen oder halbfesten Phase, insbesondere durch Verrieseln der flüssigen Phase auf einer inerten gegliederten festen Phase wie etwa einer Anhäufung von Glaskugeln, um die Oberfläche der Grenzfläche zwischen Flüssigkeit und Gas zu vergrößern.

**19.** Verwendung des Verfahrens nach einem der vorstehenden Ansprüche zur Detektion der Anwesenheit eines Mikroorganismus oder von Mikroorganismen in einen biologischen Milieu, vorzugsweise einem physiologischen Milieu.

**20.** Verwendung des Verfahrens nach einem der Ansprüche 1 bis 18 zur Ausführung eines Antibiogrammtests (AST).

**Claims**

**1.** A method for determining the presence or absence of at least one microorganism in a biological medium, said

method comprising at least the steps consisting in:

1) providing an enclosure containing:

- a liquid or semi-solid phase formed in whole or in part of said biological medium capable of containing at least one living form of said microorganism, nutritional elements necessary for proliferation of said microorganism, and an enzymatic substrate that is specific to said microorganism and that can be metabolized into at least one VOC volatile metabolite, and
- a gas phase adjacent to said liquid or semi-solid phase,

2) exposing at least said liquid or semi-solid phase to conditions that are favorable for said microorganism to metabolize said enzymatic substrate into at least one molecule of said VOC volatile metabolite, and
3) determining, by optical transduction, the presence or absence of said VOC metabolite, an indicator of the presence of said microorganism,

wherein:

- said VOC metabolite, if formed in step 2), interacts with a nanoporous matrix, said matrix being implemented in a form that is separate from said enzymatic substrate, and
- the detection by optical transduction of a change in the optical properties of said matrix indicates that said matrix interacts with said metabolite.

**2.** The method as claimed in the previous claim, wherein said metabolite has intrinsic optical properties.

**3.** The method as claimed in claim 1 or 2, wherein said VOC metabolite is distinct from natural metabolites generated during microbial growth.

**4.** The method as claimed in any one of the preceding claims, wherein said nanoporous matrix is arranged in the gas phase or liquid phase in said enclosure.

**5.** The method as claimed in any one of the preceding claims, wherein detection by optical transduction is conducted in said nanoporous matrix arranged in the gas phase.

**6.** The method as claimed in any one of the preceding claims, wherein said enzymatic substrate is a phenol or naphthol substrate.

**7.** The method as claimed in any one of the preceding claims, wherein said VOC metabolite is chosen from phenol derivatives, such as nitrophenol, cyanophenol, cyanonitrophenol, acetylphenol, propionylphenol derivatives, thiophenol derivatives, naphthol derivatives, aniline derivatives, such as nitroaniline derivatives, or naphthylamine derivatives, and in particular is chosen from p-cyanophenol, 4-nitrophenol (p-nitrophenol), m-cyano-p-nitrophenol (3-cyano-4-nitrophenol), 2,6-dichloro-4-nitrophenol, 2,6-dichloro-4-acetylphenol, thiophenol, 2,6-dichloro-4-propionylphenol, 2,6-difluoro-4-acetylphenol, 2,6-dibromo-4-acetylphenol, 1-naphthol, 2-naphthol, $\alpha$-naphthylamine, $\beta$-naphthylamine, 4-nitroaniline (p-nitroaniline), umbelliferone, naphthazarin, 4-trifluoromethylumbelliferone, 4-methylumbelliferone, o-cyanophenol, m-cyanophenol, 6-cyano-2-naphthol, 6-hydroxyquinoline-N-oxide, 2-methyl-6-hydroxyquinoline-N-oxide, 6-hydroxyquinoline, 7-hydroxyquinoline, or 8-hydroxyquinoline.

**8.** The method as claimed in any one of the preceding claims, wherein the VOC metabolite is a photoacid or a photobase.

**9.** The method as claimed in any one of the preceding claims, wherein said VOC metabolite can be detected by absorbance and/or fluorescence.

**10.** The method as claimed in any one of the preceding claims, wherein said VOC metabolite can be detected by absorbance, and in that the associated nanoporous matrix is transparent or absorbs poorly in the detection zone.

**11.** The method as claimed in any one of the preceding claims, wherein said VOC metabolite can be detected by fluorescence, and in that the associated nanoporous matrix is void of intrinsic fluorescence or has low intrinsic fluorescence.

**12.** The method as claimed in any one of the preceding claims, wherein said matrix has a pore size distribution adjusted to the size of said VOC metabolite, and in particular below 100 nm, and preferably varying from 3 to 100 Å.

**13.** The method as claimed in any one of the preceding claims wherein said matrix has a specific surface area varying from 300 to 1000 $m^2.g^{-1}$, or from 300 to 900 $m^2.g^{-1}$, and in particular from 400 to 900 $m^2.g^{-1}$.

**14.** The method as claimed in any one of the preceding claims, wherein said matrix is constituted of an organic, inorganic or organic-inorganic hybrid substance.

**15.** The method as claimed in any one of the preceding claims, wherein said matrix derives from the polycondensation of alcoholates having formula

$$M(X)_m(OR_a)_n(R_b)_p,$$

in which:

- M corresponds to a metal chosen from silicon, aluminum, tungsten, titanium, zirconium, niobium, vanadium, tantalum, yttrium and cerium,
- $R_a$ corresponds to a $C_1$ to $C_6$ alkyl radical or to a $C_5$ aryl,
- $R_b$ corresponds to a $C_1$ to $C_6$ alkyl, to a $C_5$ to $C_{10}$ aryl or to a $C_3$ to $C_6$ aminoalkyl,
- n, m and p are integers, such that their sum is equal to the valence of M and n is greater than or equal to 2, where m and p may be equal to 0, and
- X is a halogen, preferably chlorine.

**16.** The method as claimed in any one of the preceding claims, wherein said matrix comprises at least one probe molecule that can amplify the optical transduction signal via its interaction with said VOC metabolite trapped in said matrix, said probe being in particular chosen from 4-benzoylamino-2,5-diethoxybenzenediazonium chloride (Fast Blue BB), dimethylaminocinnamaldehyde (DMACA) and 5,5'-dithio-bis-(2-nitrobenzoic acid), called Ellman's reagent.

**17.** The method as claimed in any one of the preceding claims, wherein said matrix is arranged in the gas phase and in that step 2) uses means optimizing the circulation of said VOC volatile metabolite towards the gas phase.

**18.** The method as claimed in the previous claim, wherein the optimizing means are embodied by mechanical stirring or surface nebulization of the liquid or semi-solid phase, and in particular are embodied by the liquid phase flowing on a divided inert solid phase, such as an accumulation of glass beads, to increase the surface area of the liquid-gas interface.

**19.** The method as claimed in any one of the preceding claims, to detect the presence of microorganism(s) in a biological medium, and preferably in a physiological medium.

**20.** The method as claimed in any one of claims 1 to 18, to conduct an antibiogram test (AST).

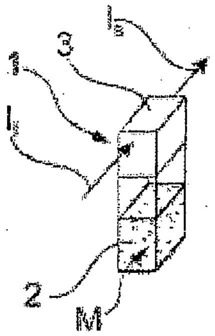


Fig. 1

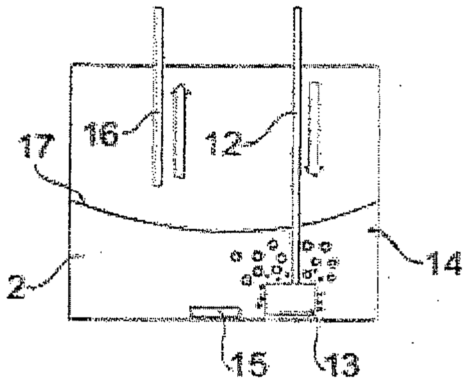


Fig. 3

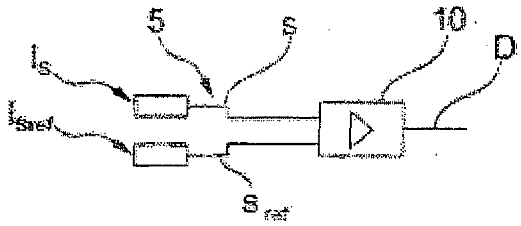


Fig. 2

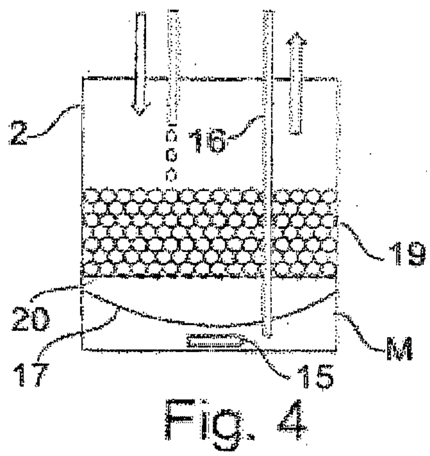


Fig. 4

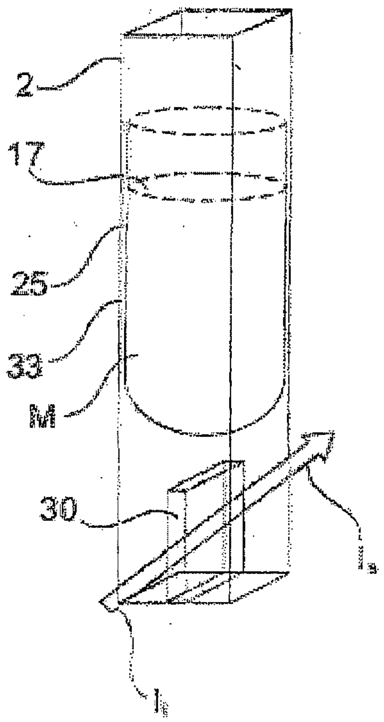


Fig. 5

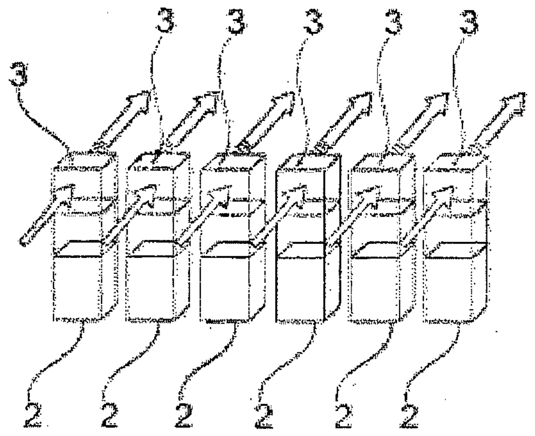


Fig. 6

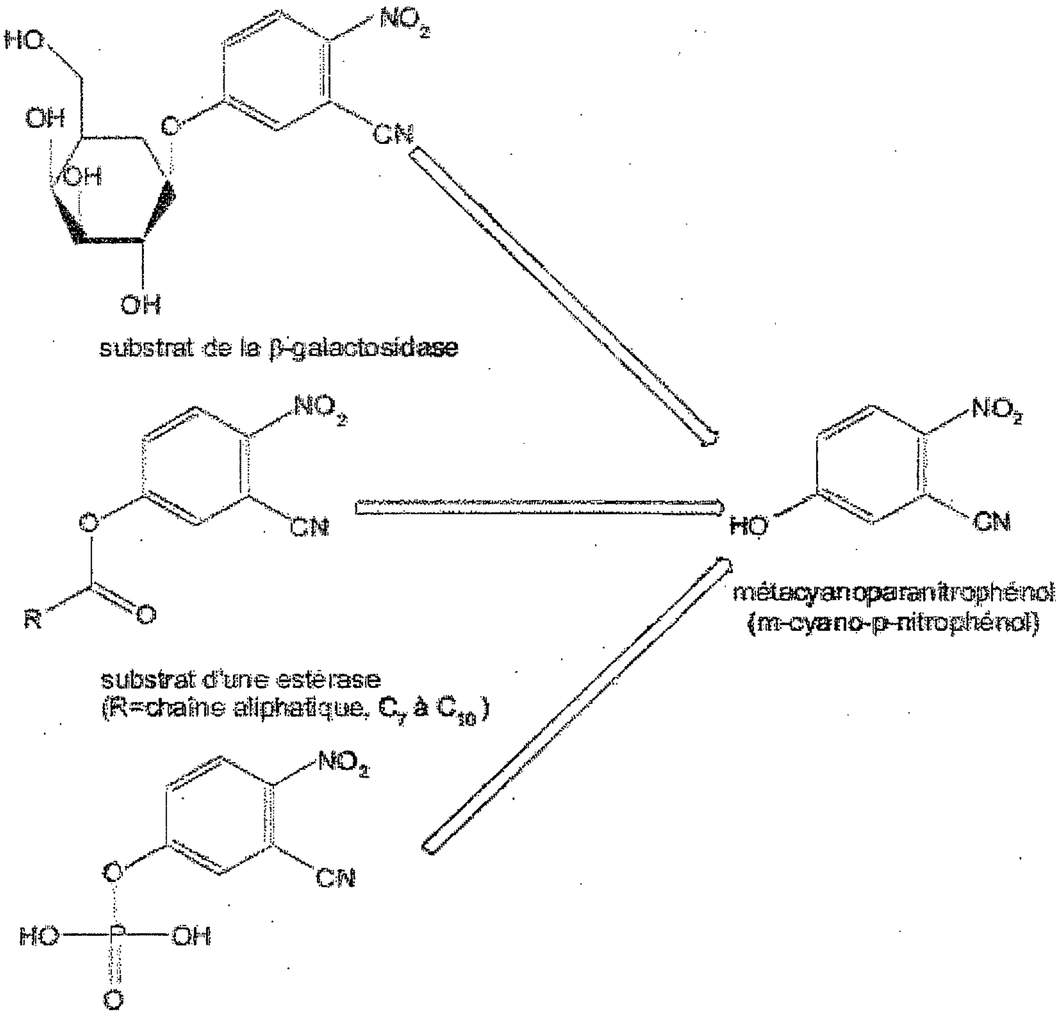

substrat de la β-galactosidase

substrat d'une estérase
(R=chaîne aliphatique, $C_7$ à $C_{10}$)

métacyanoparanitrophénol
(m-cyano-p-nitrophénol)

substrat de la phosphatase alcaline (PAL)

Fig. 7

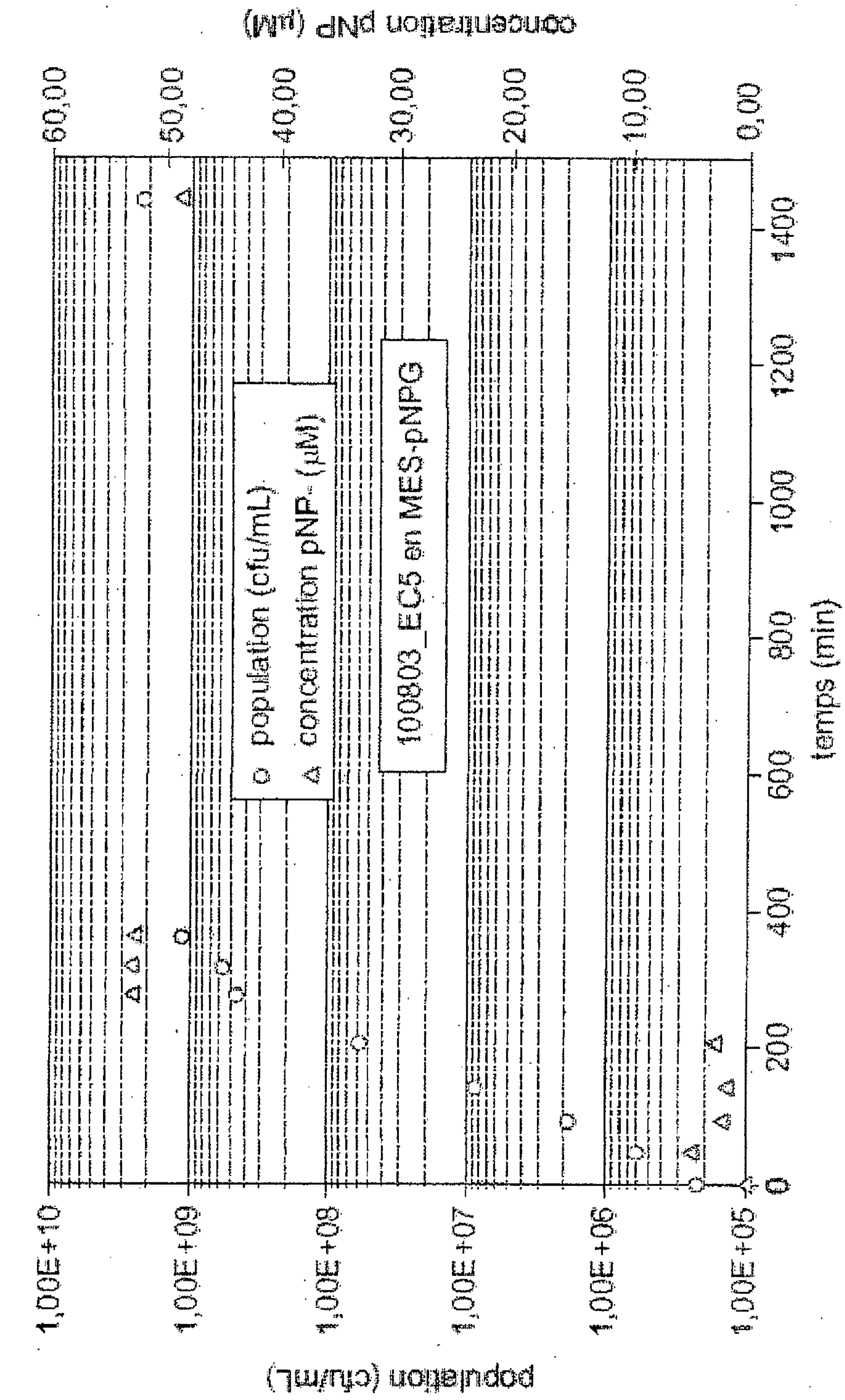

concentration pNP (µM)
60,00
50,00
40,00
30,00
20,00
10,00
0,00
o population (cfu/mL)
Δ concentration pNP- (µM)
100803_EC5 en MES-pNPG
1,00E+10
1,00E+09
1,00E+08
1,00E+07
1,00E+06
1,00E+05
population (cfu/mL)
0
200
400
600
800
1000
1200
1400
temps (min)


Fig. 8

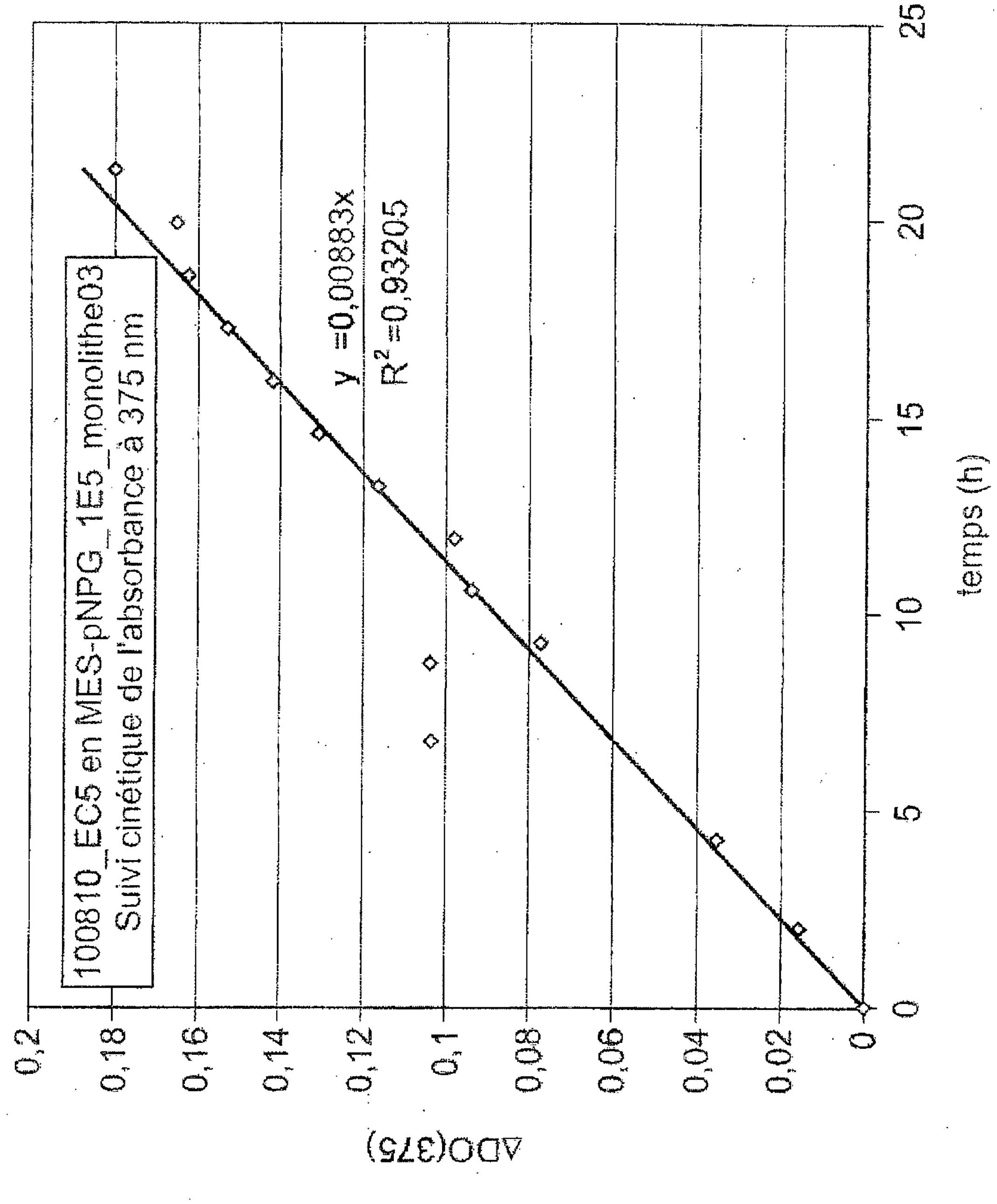
100810_EC5 en MES-pNPG_1E5_monolithe03
Suivi cinétique de l'absorbance à 375 nm
y =0,00883x
$R^2$ =0,93205
ΔDO(375)
0,2
0,18
0,16
0,14
0,12
0,1
0,08
0,06
0,04
0,02
0
0
5
10
15
20
25
temps (h)

Fig. 9

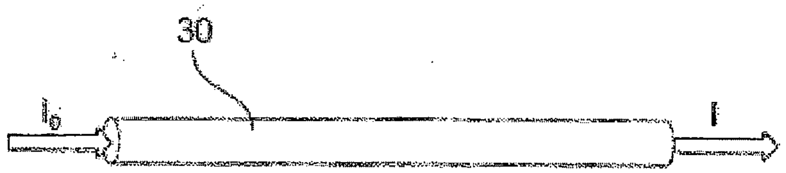


Fig. 10

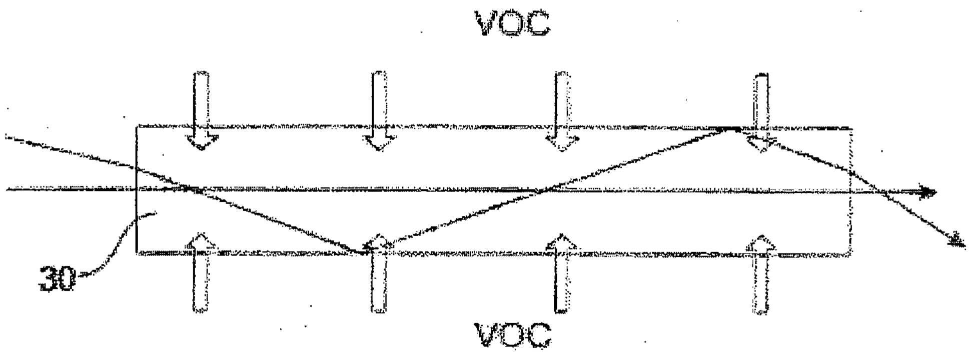


Fig. 11

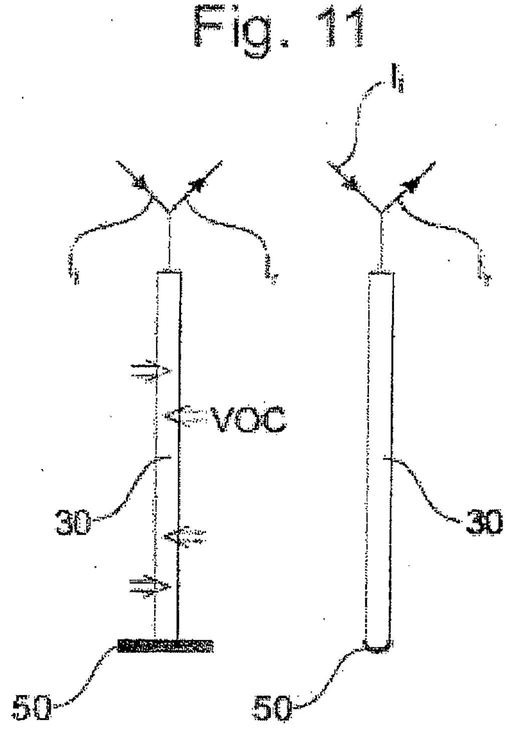


Fig. 12A Fig. 12B

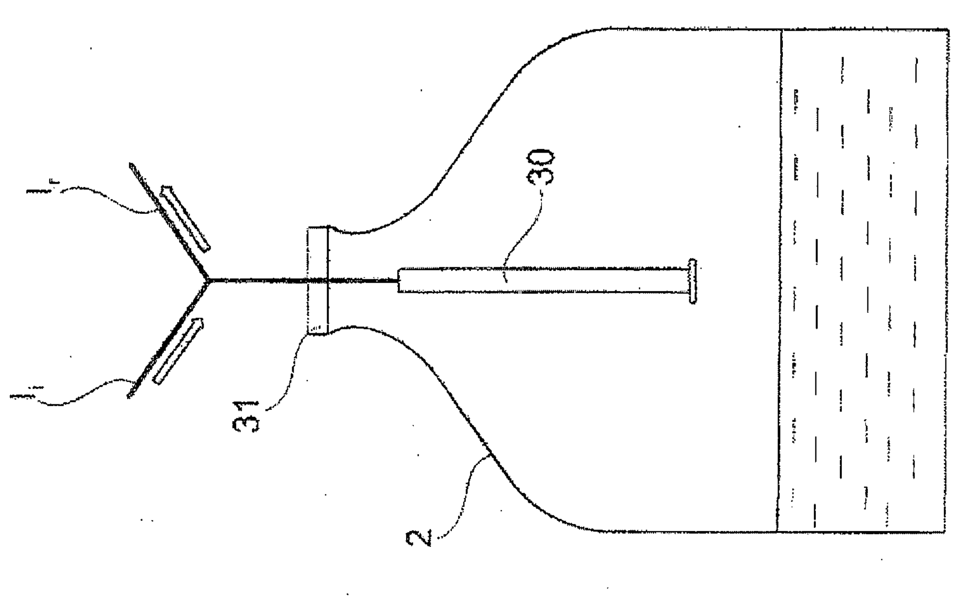


Fig. 13A

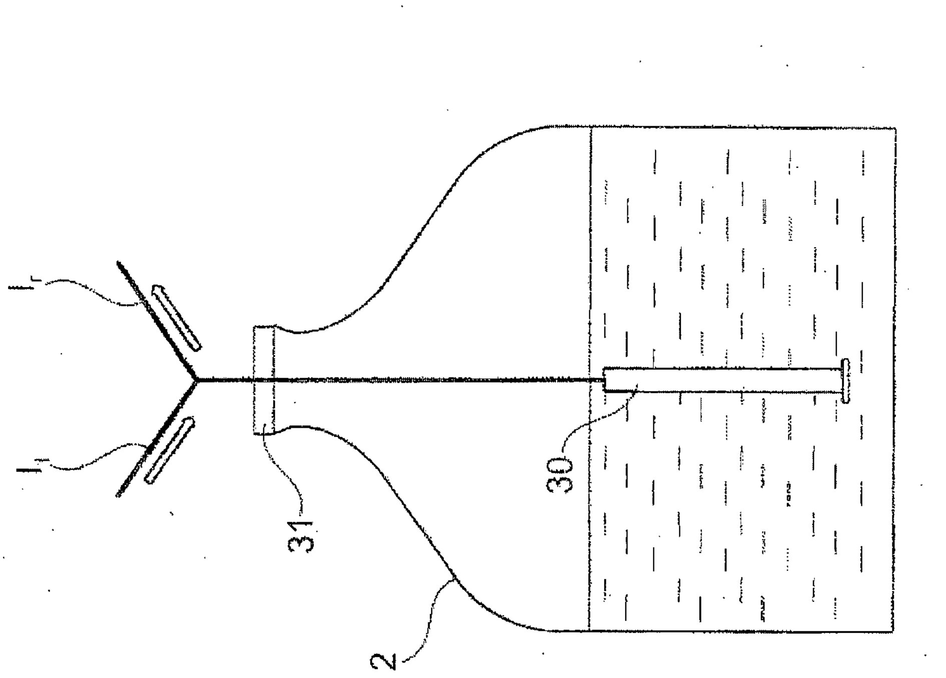


Fig. 13B

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 682252 A
- WO 2006107370 A, JG McDonald
- US 20030166298 A, K.S. Suslick
- US 20100273209 A1
- US 4945060 A, J. Turner
- US 20050171449 A
- WO 2010028057 A
- US 20080199904 A
- WO 2007031657 A
- WO 2010004225 A


### Littérature non-brevet citée dans la description


- **LOISEAU et al.** *Tetrahedron,* 2002, vol. 58, 4049-4052